# EUROPEAN PATENT SPECIFICATION

(11) **EP 3 372 074 B1**
(45) Date of publication and mention of the grant of the patent: **13.07.2022**
(21) Application number: 17206420.6
(22) Date of filing: 16.01.2017
(51) Int. Cl.: A01K 67/033, A01N 63/00, A01G 13/00, B32B 15/00

(54) **SYSTEM FOR RELEASING BENEFICIAL MITES AND USES THEREOF**
SYSTEM ZUR ABGABE VON VORTEILHAFTEN MILBEN UND VERWENDUNGEN DAVON
SYSTÈME DE LIBÉRATION D'ACARIENS UTILES ET UTILISATIONS ASSOCIÉES

(30) Priority: 15.01.2016 NL 2016103
(43) Date of publication of application: 12.09.2018
(62) Divisional of application: 17151679.2
(73) Proprietor: Koppert B.V., 2651 BE Berkel en Rodenrijs (NL)
(72) Inventor: GROOT, Thomas Volkert Marie, 2651 BE Berkel en Rodenrijs (NL); OUDE LENFERINK, Kirsten Eva Elisabeth, 2651 BE Berkel en Rodenrijs (NL); VAN HOUTEN, Yvonne Maria, 2651 BE Berkel en Rodenrijs (NL); VAN BAAL, Adelmar Emmanuel, 2651 BE Berkel en Rodenrijs (NL); HOOGERBRUGGE, Hans, 2651 BE Berkel en Rodenrijs (NL)
(74) Representative: Patentwerk B.V.

(56) References cited:
- WO-A1-2010/079353
- CN-U- 201 444 826
- GB-A- 2 393 890
- US-A1- 2005 178 337

## Description

### FIELD OF THE INVENTION

This invention in general relates to the use of mite species for human purposes. Mite species that can be used beneficially for human purposes may for example be employed to control pests, such as in the field of agriculture, including agricultural production systems for plant products, agricultural production systems for animal products, and animal husbandry, in the field of storage of food products. In such uses, predatory mite species as well as mite species suitable as prey for predatory mite species or for other predatory arthropod species may be considered beneficial.

### BACKGROUND

Within agriculture, including horticulture, the use of beneficial mites is known. For example predatory mites, such as those described in EP1686849B1, EP2042036B1, EP1830631B1, EP1965634B1 may be employed to control crop pests. EP2405741 and EP2612551B1 mention a further number of beneficial predatory mites. The areas mentioned above where mite species may be employed for human benefit encompass/include only a few of the possibilities.

For successfully employing beneficial mites the successful release of the beneficial mites in a target area is required. Various systems have been developed to release beneficial mites or to provision them with prey mites. In all these systems, beneficial mites are placed either in containers made of materials that are permeable for metabolic gasses (in particular O₂) or that have relatively large ventilation openings as to allow gas exchange with the ambient atmosphere. This on the basis of the general conviction in the art, that the prolonged survival (during at least 2 weeks) of beneficial mites in the containers requires extensive gas exchange. These requirements are amongst others reflected by GB2393890 (see e.g. page 4, line 30 - page 5, line 2) relating to a releasing system for beneficial insects and mites made of materials permeable to gas (fabric or polyethene (PE) coated paper).

However, for the prolonged release of beneficial mites, the use of systems that employ materials that are permeable for gasses and/or that have relatively large ventilation openings have certain drawbacks. In particular materials that are considerably permeable to gases also allow considerable exchange of water vapour. Similarly large ventilation openings apart from allowing exchange of metabolic gasses also allow water vapour outflow. In addition large ventilation openings impose a risk of liquid water entering the interior of the system, where the beneficial mites are present. Due to this, maintaining moisture levels within targeted ranges is a problem with the prior art systems. A moisture level outside targeted ranges may have undesired effects on the health and/or population development of the beneficial mites in the systems. Due to this, for prolonged functioning the current systems for releasing beneficial mites require an ambient relative humidity of about 70% or higher.

The inventors of the present invention have now surprisingly found that contrary to the general conviction that gas permeable materials and/or relatively large ventilation openings must be used in systems for prolonged releasing (providing) beneficial mites, it is possible to effectively maintain populations of species of beneficial mites in a compartment enclosed by a material having a low gas permeability and wherein the openings, that connect the interior of the compartment (containing the mite individuals) with the exterior, are relatively small (e.g. such as within the size range of existing systems employing gas permeable materials).

### SUMMARY

The invention therefore according to a first aspect relates to a system for releasing beneficial mites consisting of a compartment, the "mite compartment", holding a population of a beneficial mites species, preferably in association with a carrier, and a food source for the beneficial mites wherein said mite compartment is enclosed by material, gas barrier material, having a water vapour transmission rate of ≤ 5 g/m^{2∗}24 hours, said mite compartment having a volume of x mm³ of between 3^{∗}10³ to 600^{∗}10³ mm³ and wherein the system further comprises a number of connections that connect the mite compartment with the space outside the mite compartment, said number of connections each having an area y of between 0.1 and 4.0 mm², wherein the sum of the areas of the number of connections is Σy and wherein 5^{∗}10³ mm ≤ xΣy ≤ 70^{∗}10³ mm, preferably 6^{∗}10³ mm ≤ xΣy ≤60^{∗}10³ mm, more preferably 7^{∗}10³ mm ≤ x/Σy ≤ 50^{∗}10³ mm.

A further aspect of the invention relates to the use of the system according to the invention for introducing a beneficial mite species in a target area and a method for controlling in a target area a pest capable of being preyed by a predatory arthropod species said method comprising, providing the system according to the invention to said target area.

Yet another aspect of the invention relates to a method for producing an agricultural product from a number of non-human organisms susceptible to a pest capable of being preyed by a predatory arthropod species, said method comprising:
- providing the number of non-human organisms in an area, the target area;
- providing in the target area a number of systems according to the invention, such as a number of systems defined by embodiments 1-6 described below;
- providing to the number of non-human organisms suitable nutrients and environmental conditions to produce the agricultural product.

### BRIEF DESCRIPTION OF THE FIGURES

Figure 1A presents a view on the front side of a mite releasing system according to the invention.
Figure 1B presents a view on the rear side of a mite releasing system according to the invention.
Figure 1C presents a view in the direction of the longest axis of the mite releasing system presented in figures 1A and 1B.
Figure 1D presents a planar foil from which the mite rearing system of figures 1A-1C is formed.
Figure 2 shows how multiple mite releasing sachets can be formed from a roll of foil.
Figures 3A and 3B show the results of countings of predatory mites (A. *swirskii*) and prey mites (C. *lactis)* inside the mite releasing systems having the different design variations as tested in the experiment.
Figures 4A and 4B show the values of the water activity (a_{w}) and water content over time inside the mite releasing systems having the different design variations as tested in the experiment.
Figures 5A and 5B show the results of countings of predatory mites (A. *swirskii*) and prey mites (C. *lactis)* collected in a walking out test as tested in the experiment.

### DETAILED DESCRIPTION

The system of the invention is a system suitable for releasing beneficial mites. The system comprises structural elements, in particular gas barrier material and in certain embodiments also others, and biological elements, in particular the population of beneficial mites. Such a system for releasing beneficial mites may also be referred to as a device for releasing beneficial mites or a container for releasing beneficial mites.

The biological term mites will be clear to the skilled person. In particular the skilled person will know that mites are invertebrate animals from the subclass Acari characterised by having an exoskeleton and jointed appendages. The beneficial mites to be released by the system of the invention are beneficial in respect of useful functions they may perform. Such useful functions may for example include functions in agriculture, including horticulture, such as control of populations of insect and/or mite pests. In particular predatory mites are useful for the control of populations of insect and/or mite pests. Alternatively the beneficial mites may be useful in the sense that they may serve as a food source for beneficial predatory mites or other beneficial predatory arthropods, while not being a pest in the target area where they are employed. In this way they may support the development of a population of a predatory species present in the target area (either by human introduction or by being naturally present) with a minimal risk of causing negative effects in the target area. As such the term beneficial should be understood as meaning useful.

Predatory mites may for example be selected from:
- *Mesostigmatid* mite species such as from:
   i) *Phytoseiidae* such as from:
      - the subfamily of the *Amblyseiinae,* such as from the genus *Amblyseius,* e.g. *Amblyseius andersoni, Amblyseius aerialis, Amblyseius swirskii, Amblyseius herbicolus or Amblyseius largoensis,* from the genus *Euseius* e.g. *Euseius finlandicus, Euseius hibisci, Euseius ovalis, Euseius victoriensis, Euseius stipulatus, Euseius scutalis, Euseius tularensis, Euseius addoensis, Euseius concordis, Euseius ho, Euseius gallicus, Euseius citrifolius* or *Euseius citri,* from the genus *Iphiseiodes* e.g. *Iphiseiodes zuluagi,* , from the genus *Iphiseius* e.g. *Iphiseius degenerans,* from the genus *Neoseiulus* e.g. *Neoseiulus barkeri, Neoseiulus californicus, Neoseiulus cucumeris, Neoseiulus longispinosus, Neoseiulus womersleyi, Neoseiulus idaeus, Neoseiulus anonymus, Neoseiulus paspalivorus, Neoseiulus reductus* or *Neoseiulus fallacis, Neoseiulus baraki* from the genus *Amblydromalus e.g. Amblydromalus limonicus* from the genus *Typhlodromalus* e.g. *Typhlodromalus aripo, Typhlodromalus lailae* or *Typhlodromalus peregrinus* from the genus *Transeius* (alternatively known as *Typhlodromips)* e.g. *Transeius montdorensis* (alternatively known as *Typhlodromips montdorensis),* from the genus *Phytoseiulus,* e.g. *Phytoseiulus persimilis, Phytoseiulus macropilis, Phytoseiulus longipes, Phytoseiulus fragariae;*
      - the subfamily of the *Typhlodrominae,* such as from the genus *Galendromus* e.g. *Galendromus occidentalis,* from the genus *Metaseiulus* e.g. *Metaseiulus flumenis,* from the genus *Gynaeseiu* e.g. *Gynaeseius liturivorus* from the genus *Typhlodromus* e.g. *Typhlodromus exhilarates, Typhlodromus phialatus, Typhlodromus recki, Typhlodromus transvaalensis, Typhlodromus pyri, Typhlodromus doreenae* or *Typhlodromus athiasae;*
   ii) *Ascidae* such as from the genus *Proctolaelaps,* such as *Proctolaelaps pygmaeus* (Muller); from the genus *Blattisocius* e.g. *Blattisocius tarsalis* (Berlese), *Blattisocius keegani* (Fox); from the genus *Lasioseius* e.g. *Lasioseius fimetorum* Karg, *Lasioseius floridensis* Berlese, *Lasioseius bispinosus* Evans, *Lasioseius dentatus* Fox, *Lasioseius scapulatus* (Kenett), *Lasioseius athiasae* Nawar & Nasr; from the genus *Arctoseius* e.g. *Arctoseius semiscissus* (Berlese); from the genus *Protogamasellus* e.g. *Protogamasellus dioscorus* Manson;
   iii) *Laelapidae* such as from the genus *Stratiolaelaps* e.g. *Stratiolaelaps scimitus* (Womersley); *Gaeolaelaps* e.g. *Gaeolaelaps aculeifer* (Canestrini); *Androlaelaps* e.g. *Androlaelaps casalis* (Berlese), *Cosmolaelaps* e.g. *Cosmolaelaps claviger, Cosmolaelaps jaboticabalensis;*
   iv) *Macrochelidae* such as from the genus *Macrocheles* e.g. *Macrocheles robustulus* (Berlese), *Macrocheles muscaedomesticae* (Scopoli), *Macrocheles matrius* (Hull);
   v) *Parasitidae* such as from the genus *Pergamasus* e.g. *Pergamasus quisquiliarum* Canestrini; *Parasitus e.g.Parasitus fimetorum* (Berlese), *Parasitus bituberosus, Parasitus mycophilus, Parasitus mammilatus;*
- *Prostigmatid* mite species such as from:
   vi) *Tydeidae* such as from the genus *Homeopronematus* e.g. *Homeopronematus anconai* (Baker); from the genus *Tydeus e.g.Tydeus lambi* (Baker), *Tydeus caudatus* (Dugés),; from the genus *Pronematus* e.g. *Pronematus ubiquitous* (McGregor);
   vii) *Cheyletidae* such as from the genus *Cheyletus* e.g. *Cheyletus eruditus* (Schrank), *Cheyletus malaccensis* Oudemans;
   viii) *Cunaxidae* such as from the genus *Coleoscirus e.g.Coleoscirus simplex* (Ewing), from the genus *Cunaxa* e.g. *Cunaxa setirostris* (Hermann);
   ix) *Erythraeidae* such as from the genus *Balaustium* e.g. *Balaustium putmani* Smiley, *Balaustium medicagoense* Meyer &Ryke , *Balaustium murorum* (Hermann), *Balaustium hernandezi, Balaustium leanderi;*
   x) *Stigmaeidae* such as from the genus *Agistemus* e.g. *Agistemus exsertus* Gonzalez; such as from the genus *Zetzellia* e.g. *Zetzellia mali* (Ewing);
   xi) *Anystidae,* such as from the genus *Anystis,* e.g. *Anystis baccarum.*

In view of their predatory behaviour towards important pests, predatory mites preferably are selected from the family Phytoseiidae, in particular from the genus *Amblyseius,* such as *Amblyseius swirskii, Amblyseius largoensis* and *Amblyseius andersoni,* from the genus *Neoseiulus,* such as *Neoseiulus californicus, Neoseiulus cucumeris, Neoseiulus barkeri, Neoseiulus baraki* and *Neoseiulus longispinosus* and *Neoseiulus fallacis,* in particular from the genus *Euseius,* such as *Euseius gallicus,* in from the genus *Iphiseius,* such as *Iphiseius degenerans,* from the genus *Transeius,* such as *Transeius montdorensis,* from the genus *Amblydromalus,* such as *Amblydromalus limonicus* (alternatively known as *Typhlodromalus limonicus),* from the genus *Galendromus,* such as *Galendromus occidentalis,* from the genus *Phytoseiulus,* such as *Phytoseiulus persimilis, Phytoseiulus macropilis* and *Phytoseiulus longipes,* from the family Cheyletidae, in particular from the genus *Cheyletus,* such as *Cheyletus eruditus,* from the family Laelapidae, in particular from the genus *Androlaelaps,* such as *Androlaelaps casalis,* from the genus *Stratiolaelaps,* such as *Stratiolaelaps scimitus* (Alterntively known as *Hypoaspis miles),* from the genus *Gaeolaelaps,* such as *Gaeolaelaps aculeifer* (Alternatively known as *Hypoaspis aculeifer),* or from the family Macrochelidae, in particular from the genus *Macrocheles,* such as *Macrocheles robustulus.*

The names of the Phytoseiidae are as referred to in Chant D.A., McMurtry, J.A. (2007) Illustrated keys and diagnoses for the genera and subgenera of the Phytoseiidae of the world (Acari: Mesostigmata), Indira Publishing House, West Bloomfied, MI, USA. The names of the Ascidae, the Laelapidae, the Macrochelidae, the Parasitidae, the Tydeidae, the Cheyletidae, the Cunaxidae, the Erythraeidae and the Stigmaeidae are as referred to in Carrillo, D., de Moraes, G.J., Pena, J.E. (ed.) (2015) Prospects for Biological Control of Plant Feeding Mites and Other Harmful Organisms. Springer, Cham, Heidelberg, New York, Dordrecht, London. For *Parasitus mycophilus* reference may be made to Baker A.S.,Ostoja-Starzewski J.C (2002) New distributional records of the mite Parasitus mycophilus (Acari: Mesostigmata), with a redescription of the male and first description of the deutonymph. Systematic & Applied Acarology 7, 113-122. For *Parasitus* mammilatus refrence may be made to Karg, W. (1993) Die Tierwelt Deutschlands, 59.Teil. Acari (Acarina), Milben Parasitiformes (Anactinochaeta) Cohors Gamasina Leach. Gustav Fischer, Jena. For the Anystidae reference may be made to Cuthbertson A.G.S., Qiu B.-L., Murchie A.K. (2014) Anystis baccarum: An Important Generalist Predatory Mite to be Considered in Apple Orchard Pest Management Strategies. Insects 5, 615-628; doi:10.3390/insects5030615.

The skilled person will know the potential host range of the selected predatory mite species. Pests that may be effectively controlled with predatory mites are for example white flies, such as *Trialeurodes vaporariorum* and *Bemisia tabaci;* thrips, such as *Thrips tabaci, Thrips palmi* and *Frankliniella spp.,* such as *Frankliniella occidentalis, Frankliniella schultzei* spider mites such as *Tetranychus urticae, Panonychus ulmi,* other phytophagous mites such as *Polyphagotarsonemus latus,* or other pest such as Eriophyids, Tenuipalpids, Psyllids, leafhoppers, aphids, diptera. In addition mites infesting avian species, such as the red poultry mite *(Dermanyssus gallinae)* and mites infesting reptiles, such as from the family Macronyssidae, such as from the genus *Ophionyssus,* such as *Ophionyssus natricis,* may also be preyed by predatory mites, in particular predatory mites selected from the genus *Hypoaspis,* such as *Hypoaspis angusta,* from the genus *Cheyletus,* such as *Cheyletus eruditis,* from the genus *Androlaelaps,* such as *Androlaelaps casalis,* from the family *Laelapidae* such as from the genus *Stratiolaelaps* e.g. *Stratiolaelaps scimitus* (Womersley); *Gaeolaelaps* e.g. *Gaeolaelaps aculeifer* (Canestrini); *Androlaelaps* e.g. *Androlaelaps casalis* (Berlese), or from the genus *Macrocheles,* such as *Macrocheles robustulus.*

Beneficial mites that may serve as a food source for predatory mites or other predatory arthropods according to certain embodiments of the invention may be selected from Astigmatid mites species, in particular Astigmatid mite species selected from:
i) *Carpoglyphidae* such as from the genus *Carpoglyphus* e.g. *Carpoglyphus lactis;*
ii) *Pyroglyphidae* such as from the genus *Dermatophagoides* e.g. *Dermatophagoides pteronysinus, Dermatophagoides farinae;* from the genus *Euroglyphus* e.g. *Euroglyphus longior, Euroglyphus maynei;* from the genus *Pyroglyphus* e.g. *Pyroglyphus africanus;*
iii) *Glycyphagidae* such as from the subfamily *Ctenoglyphinae,* such as from the genus *Diamesoglyphus* e.g. *Diamesoglyphus intermediusor* from the genus *Ctenoglyphus,* e.g. *Ctenoglyphus plumiger, Ctenoglyphus canestrinii, Ctenoglyphus palmifer;* the subfamily *Glycyphaginae,* such as from the genus *Blomia,* e.g. *Blomia freemani* or from the genus *Glycyphagus,* e.g. *Glycyphagus ornatus, Glycyphagus bicaudatus, Glycyphagus privatus, Glycyphagus domesticus,* or from the genus *Lepidoglyphus* e.g. *Lepidoglyphus michaeli, Lepidoglyphus fustifer, Lepidoglyphus destructor,* or from the genus *Austroglycyphagus,* e.g. *Austroglycyphagus geniculatus;* from the subfamily *Aeroglyphinae,* such as from the genus *Aeroglyphus,* e.g. *Aeroglyphus robustus;* from the subfamily *Labidophorinae,* such as from the genus *Gohieria,* e.g. *Gohieriafusca;* or from the subfamily *Nycteriglyphinae* such as from the genus *Coproglyphus,* e.g. *Coproglyphus stammeri* or from the subfamily *Chortoglyphidae,* such as the genus *Chortoglyphus* e.g. *Chortoglyphus arcuatus* and more preferably is selected from the subfamily *Glycyphaginae,* more preferably is selected from the genus *Glycyphagus* or the genus *Lepidoglyphus* most preferably selected from *Glycyphagus domesticus or Lepidoglyphus destructor;*
iv) *Acaridae* such as from the genus *Tyrophagus* e.g. *Tyrophagus putrescentiae, Tyrophagus tropicus,* from the genus *Acarus* e.g. *Acarus siro, Acarus farris, Acarus gracilis;* from the genus *Lardoglyphus* e.g. *Lardoglyphus konoi,* from the genus *Thyreophagus,* such as *Thyreophagus entomophagus;* from the genus *Aleuroglyphus,* e.g. *Aleuroglyphus ovatus;*
v) *Suidasiidae* such as from the genus *Suidasia,* such as *Suidasia nesbiti, Suidasia pontifica or Suidasia medanensis.*

Preferred *Astigmatid* mites may be selected from *Lepidoglyphus destructor, Carpoglyphidae* such as from the genus *Carpoglyphus* e.g. *Carpoglyphus lactis,* the genus *Thyreophagus,* such as *Thyreophagus entomophagus, Acaridae,* such as *Suidasia pontifica or Suidasia medanensis.*

*Astigmatid* mites can be isolated from their natural habitats as described by Hughes (Hughes, A.M., 1977, The mites of stored food and houses. Ministry of Agriculture, Fisheries and Food, Technical Bulletin No. 9: 400 pp), and can be maintained and cultured as described by Parkinson (Parkinson, C.L., 1992, "Culturing free living astigmatid mites." Arachnida: Proceedings of a one day symposium on spiders and their allies held on Saturday 21st November 1987 at the Zoological Society of London) and by Solomon & Cunnington (Solomon, M.E. and Cunnington, A.M., 1963, Rearing acaroidmites, Agricultural Research Council, Pest Infestation Laboratory, Slough, England, pp 399 403).

The term "releasing" should be understood as meaning that beneficial mites may emerge from the system. Thus the mite releasing system of the invention is suitable for releasing, dispersal or providing beneficial mites.

The system of the invention comprises a compartment, the mite compartment, holding a population of beneficial mites. A function of the compartment is to hold the individuals of the population of the beneficial mites and any additional materials associated with the beneficial mite individuals. Such additional materials may be selected from carrier materials and/or food sources known to the skilled person.

The size and shape (or form) of the compartment may vary depending on the selected beneficial mite. Selection of suitable size ranges and shapes (or forms) is within the common knowledge of the skilled person. For example reference may be made to GB2393890 and GB2509224 disclosing systems for mites or insects having suitable shapes and sizes. The skilled person will understand that the systems according to the present invention may also be designed in correspondence with the mite releasing systems as disclosed in GB2393890 and GB2509224. The mite releasing system of the invention may therefore be in association with at least one other system of the invention by being connected to the at least one other system, thus forming an association of a plurality of systems of the invention. The association of the plurality of systems of the invention preferably is such that an elongated body is formed. The elongated body preferably has a length longer than an individual system and a breadth essentially as broad as a single system. According to certain preferred embodiments the association of systems comprises 2 systems of the invention foldable to an inverted V or U, wherein the connections are located inside the folded conformation. According to other preferred embodiments the association of systems has an elongated body at least 10 - 180 metres in length, such as 80-160 metres.

The population of beneficial mites contained in the compartment preferably is a breeding population. In this specification the term "breeding" must be understood to include the propagation and increase of a population by means of reproduction. The skilled person will know and understand that although many mite species reproduce via sexual reproduction, some species reproduce via asexual reproduction. The skilled person will be able to identify which mite species reproduce sexually and which mite species reproduce asexually. In essence a breeding population is capable of increasing the number of its individuals by means of reproduction. The skilled person will thus understand that a breeding population will comprise female mite individuals that are capable to reproduce, i.e. that can produce off spring, or female mite individuals that can mature to a life stage wherein they can produce off spring. The skilled person will further understand that for a mite species that reproduces sexually a breeding population comprises sexually mature male individuals or male individuals that may mature to sexually mature male individuals. Alternatively for a mite species reproducing sexually a breeding population may comprise one or more fertilized females..

The population of the mites preferably is in association with a carrier. The use of carriers in products comprising beneficial mites is common practice within the art and it is known that in principle any solid material which is suitable to provide a carrier surface to the individuals may be used. Therefore, in general the carrier particles will have a size larger than the size of the individuals of the beneficial mites. Preferably the carrier provides a porous medium, which allows exchange of metabolic gases and heat produced by the mite populations. The skilled person will know that the suitability of a particular carrier will depend on the species of the beneficial mite selected and will be able to select suitable carriers. For example suitable carriers may be selected from plant materials such as (wheat) bran, saw dust, corn cob grits etcetera. WO2013/103295 further discloses the suitability of chaff as a carrier material for mite populations. When a carrier is present in the mite compartment, the carrier material preferably does not fill the mite compartment completely, but there is some head space left in the mite compartment. Head space may be created by using a carrier volume of 60-95%, preferably 70-90%, more preferably 75-85% of the volume x of the mite compartment. Head space may contribute to gas exchange via the number of connections. In view of this, in case a carrier is used and there is head space in the mite compartment, the number of connections preferably are provided in the upper part of the mite compartment (where the head space will be located).

The compartment further comprises a food source for the beneficial mites. The skilled person will know that the suitability of a food source may depend on the selected species of the beneficial mite. For predatory species a living prey may be preferred. For example Astigmatid mites may be suitable prey for predatory mites. Astigmatid mite species that may be selected as food source for predatory mite species are already indicated above. Thus according to certain embodiments of the invention the mite compartment may comprise a predatory mite species as the beneficial mite and an astigmatid mite species as a food source for the predatory mite. According to further embodiments of the invention the population of Astigmatid mite species presented as a food source for the predatory mite may be at least partially be immobilized as disclosed in WO2013/103294. In addition eggs from the lepidopterans *Corcyra cephalonica* or *Ephestia kuehniella* may be suitable as a food source for many mesostigmatid or prostigmatid predatory mites, such as phytoseiid predatory mites. As the skilled person will know, lepidopteran eggs are usually inactivated, when presented as as a food source to predatory mites. The skilled person will know that further food sources for predatory mites may be selected from Artemia or from pollen, such as pollen of *Typha* spp..

The mite compartment of the system of the invention is enclosed by material, having a low gas exchange rate and in particular a water vapour transmission rate of ≤ 5 g/m^{2∗}24 hours. Materials with such low water vapour transmission rates also have low transmissions rates for metabolic gasses produced by the mites (and microorganisms also present in mite cultures) such as O₂ and/or CO₂. As is already indicated above, the inventors of the present invention have now surprisingly found that contrary to the general conviction that gas permeable materials must be used in systems for releasing (providing) beneficial mites, it is possible to effectively maintain populations of species of beneficial mites in a compartment enclosed by a material having a low gas permeability. Any material having the indicated water vapour transmission rate may be suitably employed within the present invention. There is no particular lower limit for the water vapour transmission rate other than wat is technically feasible. The skilled person will know that water vapour barrier materials are available that have an infinitely small water vapour transmissions rate. Thus the water vapour transmission rate of a selected gas barrier material may be between 5.0 g/m^{2∗}24 hours and the theoretical value of 0.00 g/m^{2∗}24 hours. Suitable gas barrier materials may have a water vapour transmission rate between 5.0-0.01 g/m^{2∗}24 hours, preferably between 3.5-0.05 g/m^{2∗}24 hours, such as between 2.5-0.1 g/m^{2∗}24 hours, more preferably between 2.0-1.0 g/m^{2∗}24 hours, such as between 2.0-0.5 g/m^{2∗}24 hours, most preferably between 2.0-1.0 g/m^{2∗}24 hours.

The skilled person will understand that any connections made between different parts of gas barrier material required to create the mite compartment must also be resistant to water vapour transmission in the same range as the gas barrier material. The skilled person will have knowledge how to make connections resistant to water vapour transmission. Suitable gas barrier materials preferably will further allow the creation of seals that are resistant to water vapour transmission.

Within the present description the term "compartment" refers to a part or space that is partitioned off. In the system of the present invention the space of the mite compartment is partitioned off by being enclosed by gas barrier material. The reference to the mite compartment being "enclosed" by gas barrier material thus means that the compartment space is surrounded by (or enveloped in) gas barrier material. Gas barrier material used, preferably is in sheet form, more preferably pliable sheet. The mite compartment is enclosed by a number of planes of gas barrier material. For enclosing, surrounding, enveloping the mite compartment, a "number of" gas barrier materials is used. Preferably a single type of gas barrier material is used for all planes of gas barrier material enclosing the mite compartment, such that the "number of" gas barrier materials refers to a gas barrier material, i.e. the singular. However, in certain alternative embodiments different types of gas barrier materials may be used for different planes within the total of planes enclosing the mite compartment. For example in a sachet a front plane may be from and first gas barrier material and a back plane may be from a second type of gas barrier material. In such cases the number of gas barrier materials refers to a plurality of gas barrier materials.

The term "plane" refers to a surface with any possible shape or configuration. Preferably the number of planes enclosing the mite compartment are at least essentially flat. Alternatively the planes may be curved. According to certain embodiments the planes may be of a mixed form including areas that are at least essentially flat and areas that are curved. At least essentially flat includes flat and perfectly flat.

"A number of" within this description of the present invention means one or more, such as 1, 2, 3, 4, 5, 6, 7, 8, 9, 10 or more. In certain embodiments a number of is a plurality such as 2, 4, 5, 6, 8 or 10. The number of planes of gas barrier material enclosing the mite compartment may be a single plane. The skilled person will know and will understand that a single plane can form a 3-dimensional enclosure enclosing a compartment having a certain volume, if a plane is bend and fixed in a 3-dimensional enclosing structure. For example a closed compartment in shape similar to a sugar stick or coffee creamer stick may be formed from a rectangular pliable sheet bent in a cylindrical shape and fixing the sides meeting at the cylinder mantle to form a closed mantle and subsequently fixing the two opposing open ends (on "top" and "bottom" end) of the cylinder to close the open ends. The compartment in such an object is enclosed by a single plane of the enclosing sheet.

The skilled person will have knowledge as to what water vapour is and in particular that it is the gaseous state of water. The materials having the low gas exchange rate, the gas barrier materials, that are suitable for use within the present invention have a water vapour transmission rate of ≤ 5 g/m^{2∗}24 hours. According to certain preferred embodiments the test conditions for the water vapour transmission rates are 38 °C, 90% RH. Water vapour transmission rates may be determined in accordance with the procedures of the ASTM E96, the ASTM E398, or the ASTM F1249 standard. According to certain preferred embodiments, the procedures of ASTM E96 are used for determining the water vapour transmission rate. Materials having the low values of water vapour transmission as selected in the present invention also have low levels of transmission of metabolic gasses. For example the BUI43 foil (obtainable from Euroflex B.V., Zwolle, The Netherlands) according to the supplier has an oxygen permeability of about 5 cc/m^{2∗}24 hours (Measured according to ASTM F 1927 at 23 °C, 50% RH). Similarly the Nativia^{™} NZSS films (Taghleef Industries) according to the manufacturer have an oxygen permeability of about 12 cc/m^{2∗}24 hours (Measured according to ASTM D 3985 at 23 °C, 50% RH) and the EcoMet films (Ultimet Films) according to the manufacturer have an oxygen permeability of about 3.0 cc/m^{2∗}24 hours (Measured according to ASTM D 3985 at 23 °C, 50% RH).

Selection of a gas barrier material may be from any material having the indicated water vapour transfer rate and the skilled person will be able to select materials having a water vapour transfer rate within the indicated ranges. Multilayer laminates are preferred. A multilayer laminate should be understood as a laminate having at least 3 layers. Multilayer laminates in particular may have good gas barrier properties. According to certain preferred embodiments a selected gas barrier material may be a polymer-metal laminate, preferably a polymer-metal laminate film, such as a laminate film comprising a metalized polymer film. Polymer-metal laminates in particular have good gas barrier properties, in particular in case they are multi-layered. Pliable films have a particular preference as they may be more easily formed in desired shapes. A gas barrier material may for example be selected from the NatureFlex^{™} N932 (Innovia^{™} Films) film, according to the supplier having a water vapour transmission rate of < 5 g/m^{2∗}24 hours (determined according to ASTM E96 at 38 °C, 90% RH). However, observations made by the inventors indicate that this material may have a lower water vapour transmission rate than indicated by the supplier. Alternatively the BUI43 foil (obtainable from Euroflex B.V., Zwolle, The Netherlands) may be used. This BUI43 foil according to the supplier has a water vapour transmission rate of < 1.5 g/m^{2∗}24 hours (determined according to ASTM E96 at 38 °C, 90% RH). Other alternative gas barrier materials may be selected from the Nativia^{™} NZSS films (Taghleef Industries) that according to the supplier have a water vapour transmission rate of about 2.3 g/m^{2∗}24 hours (determined according to ASTM F1249 at 38 °C, 90% RH) and the EcoMet films (Ultimet Films) that according to the supplier have a water vapour transmission rate of about 1.0 g/m^{2∗}24 hours (determined according to ASTM F1249 at 38 °C, 90% RH). Although the use of these materials is particularly preferred, from the contents of this description of the invention it will be clear for the skilled person that a material other than a polymer-metal laminate, such as a polymer-metal laminate film, such as a laminate film comprising a metalized polymer film, may be selected as the gas barrier material.

The number of planes of gas barrier material enclosing the mite compartment will have a certain surface area z expressible in mm². The surface area referred to is the effective surface area of the barrier material that is the surface area defining (or forming the limits of) the mite compartment. This is the surface area of the gas barrier material that is in contact with the interior space of the mite compartment. Depending on the specific use of the mite dispensing system, the value z of the surface area of the barrier material may have a value selected from 0.5^{∗}10³-30^{∗}10³ mm², preferably 2.5^{∗}10³-15^{∗}10³ mm², more preferably 3.0^{∗}10³-7.0^{∗}10³ mm².

The mite compartment will have a certain volume x expressible in mm³. The volume of the mite compartment is the volume of the space enclosed by the planes of gas barrier material. The value x of the volume may be selected within the range of 3^{∗}10³ to 600^{∗}10³ mm³, preferably 6^{∗}10³ to 300^{∗}10³ mm³, more preferably 8^{∗}10³ to 100^{∗}10³ mm³, most preferably 9^{∗}10³ to 35^{∗}10³ mm³.

The system further comprises a number of connections that connect the interior space of the mite compartment with the space outside the mite compartment. The connections primarily have the functions of allowing gas exchange and to allow (mobile) individuals of the beneficial mite population to exit from the mite compartment. A number of should be construed as one or more as defined above. Openings in the gas barrier material are suitable to serve as connections. Openings may be provided by any suitable means known to the skilled person, such as mechanical puncturing, such as punching or needle puncturing or, when the gas barrier material has a relatively low melting temperature (below 150°C), such as is the case for many metallised polymer films, by heat puncturing or burning. Other alternative means for creating the openings may comprise laser puncturing. Preferably a method is selected that creates openings by removal of the gas barrier material.

The number of connections each will have a certain surface area y expressible in mm². The area y of a connection is the area available for gas exchange via that connection. Σy is the summation of the area of the individual connections in the system. For example in case a system of the invention comprises 2 connections, a first having an area yl of 1.0 mm² and a second having an area y2 of 2.0 then Σy = y1+y2= 1.0+2.0 = 3.0 mm². The surface area y of individual connections may have a value selected from 0.10-4.0 mm², preferably 0.15-2.0 mm², more preferably 0.20-1.5 mm², most preferably 0.20-0.50 mm². Within the indicated size ranges the shape of the connections used is such that passage of mobile mite individuals present in the mite compartment is possible through at least one of the number of connections provided. Within the broader ranges provided the skilled person will be able to select the narrower range suitable for a selected beneficial mite. Circular connections of the indicated sizes in general will be suitable for most beneficial mites. Connections of different non-circular shapes may also be suitable. Preferably non-circular connections have a shape and size that can enclose a circle having a surface area within the range mentioned for the value of y.

According to certain embodiments of the invention, the use of a plurality of connections is preferred. In case a plurality of connections is used, the number of connections may be 1 per volume fraction of the mite compartment. For example 1 per 3^{∗}10³ mm³ or alternatively 1 per 5^{∗}10³, 10^{∗}10³, 15^{∗}10³, 20^{∗}10³, 25^{∗}10³, 30^{∗}10³, 35^{∗}10³, 40^{∗}10³ or 50^{∗}10³ mm³ of volume of the mite compartment. For example for a mite compartment having a volume x of 200^{∗}10³ mm³, a plurality of connections may be provided such that 1 connection is provided per 20^{∗}10³ mm³. In this case 200/20 = 10 connections will be provided. Alternatively for a mite compartment having a volume x of 70^{∗}10³ mm³, a plurality of connections may be provided such that 1 connection is provided per 25^{∗}10³ mm³. In this case 2 connections are provided in view of the fact that 70/25 = 2,8 and the total number of connections that may be provided is 2. In general when using mite compartments having a volume x greater that 20^{∗}10³ mm³, the use of a plurality of connections is preferred.

According to certain embodiments, the connections preferably are provided at an end of the system that is an upper part. Reference to an upper part refers to the situation of use of the system of the invention. In case the system of the invention is provided with means for hanging it, the upper part will be at the end of the hanging means.

In the system of the invention, the value x of the volume of the mite compartment and the value y of the area of the connections is selected such that 5^{∗}10³ mm ≤ x/Σy ≤ 70^{∗}10³ mm, preferably 6^{∗}10³ mm ≤ x/Σy ≤ 60^{∗}10³ mm, more preferably 7^{∗}10³ mm ≤ xΣy ≤ 50^{∗}10³ mm, wherein Σy is the summation of the areas y of the connections. This assures that the openings are relatively small in comparison to the size of the compartment, thus limiting the escape of water vapour from the mite compartment. It is surprising that populations of mites can be effectively maintained in a closed compartment enclosed by a material having a low oxygen transmission and connected only with the exterior with connections of such a relatively small size.

In the system according to the invention (i) the water vapour transmission rate of the material enclosing the mite compartment (WVTR), the volume x of the mite compartment, the area y of the connections, and the fraction xΣy (wherein Σy is the total area of the connections (the summation of the area y of the individual connections)) must be within certain predefined ranges. Selections within the ranges presented must be made such that the criteria for WVTR, x, y and xΣy are all within the specified ranges. In Table I below combinations of WVTR, x, y and xΣy envisaged within the present invention are presented. In the various columns relating to different values for the WVTR, different combinations of x, y and xΣy are presented. An embodiment with particular preference has the following combinations: WVTR = 2.0-1.0 g/m^{2∗}24 hours, x = 9^{∗}10³-35^{∗}10³ mm³, y = 0.20-0.50 mm², x/Σy = 7^{∗}10³ - 50^{∗}10³ mm. *

**Table I**

| WVTR (g/m^{2∗}24h) 5.0-0.00 | WVTR (g/m^{2∗}24h) 5.0-0.01 | WVTR (g/m^{2∗}24h) 3.5-0.05 | WVTR (g/m^{2∗}24h) 2.5-0.1 | WVTR (g/m^{2∗}24h) 2.0-0.1 | WVTR (g/m^{2∗}24h) 2.0-0.5 | WVTR (g/m^{2∗}24h) 2.0-1.0 |
|---|---|---|---|---|---|---|
| x: 3k-600k | x: 3k-600k | x: 3k-600k | x: 3k-600k | x: 3k-600k | x: 3k-600k | x: 3k-600k |
| y: 0.10-4.0 | y: 0.10-4.0 | y: 0.10-4.0 | y: 0.10-4.0 | y: 0.10-4.0 | y: 0.10-4.0 | y: 0.10-4.0 |
| x/Σy: 5k-70k | x/Σy: 5k-70k | x/Σy: 5k-70k | x/Σy: 5k-70k | x/Σy: 5k-70k | x/Σy: 5k-70k | x/Σy: 5k-70k |
| x: 6k-300k | x: 6k-300k | x: 6k-300k | x: 6k-300k | x: 6k-300k | x: 6k-300k | x: 6k-300k |
| y: 0.10-4.0 | y: 0.10-4.0 | y: 0.10-4.0 | y: 0.10-4.0 | y: 0.10-4.0 | y: 0.10-4.0 | y: 0.10-4.0 |
| x/Σy: 5k-70k | x/Σy: 5k-70k | x/Σy: 5k-70k | x/Σy: 5k-70k | x/Σy: 5k-70k | x/Σy: 5k-70k | x/Σy: 5k-70k |
| x: 8k-100k | x: 8k-100k | x: 8k-100k | x: 8k-100k | x: 8k-100k | x: 8k-100k | x: 8k-100k |
| y: 0.10-4.0 | y: 0.10-4.0 | y: 0.10-4.0 | y: 0.10-4.0 | y: 0.10-4.0 | y: 0.10-4.0 | y: 0.10-4.0 |
| x/Σy: 5k-70k | x/Σy: 5k-70k | x/Σy: 5k-70k | x/Σy: 5k-70k | x/Σy: 5k-70k | x/Σy: 5k-70k | x/Σy: 5k-70k |
| x: 9k-35k | x: 9k-35k | x: 9k-35k | x: 9k-35k | x: 9k-35k | x: 9k-35k | x: 9k-35k |
| y: 0.10-4.0 | y: 0.10-4.0 | y: 0.10-4.0 | y: 0.10-4.0 | y: 0.10-4.0 | y: 0.10-4.0 | y: 0.10-4.0 |
| x/Σy: 5k-70k | x/Σy: 5k-70k | x/Σy: 5k-70k | x/Σy: 5k-70k | x/Σy: 5k-70k | x/Σy: 5k-70k | x/Σy: 5k-70k |
| x: 3k-600k | x: 3k-600k | x: 3k-600k | x: 3k-600k | x: 3k-600k | x: 3k-600k | x: 3k-600k |
| y: 0.15-2.0 | y: 0.15-2.0 | y: 0.15-2.0 | y: 0.15-2.0 | y: 0.15-2.0 | y: 0.15-2.0 | y: 0.15-2.0 |
| x/Σy: 5k-70k | x/Σy: 5k-70k | x/Σy: 5k-70k | x/Σy: 5k-70k | x/Σy: 5k-70k | x/Σy: 5k-70k | x/Σy: 5k-70k |
| x: 3k-600k | x: 3k-600k | x: 3k-600k | x: 3k-600k | x: 3k-600k | x: 3k-600k | x: 3k-600k |
| y: 0.20-1.5 | y: 0.20-1.5 | y: 0.20-1.5 | y: 0.20-1.5 | y: 0.20-1.5 | y: 0.20-1.5 | y: 0.20-1.5 |
| x/Σy: 5k-70k | x/Σy: 5k-70k | x/Σy: 5k-70k | x/Σy: 5k-70k | x/Σy: 5k-70k | x/Σy: 5k-70k | x/Σy: 5k-70k |
| x: 3k-600k | x: 3k-600k | x: 3k-600k | x: 3k-600k | x: 3k-600k | x: 3k-600k | x: 3k-600k |
| y: 0.20-0.50 | y: 0.20-0.50 | y: 0.20-0.50 | y: 0.20-0.50 | y: 0.20-0.50 | y: 0.20-0.50 | y: 0.20-0.50 |
| x/Σy: 5k-70k | x/Σy: 5k-70k | x/Σy: 5k-70k | x/Σy: 5k-70k | x/Σy: 5k-70k | x/Σy: 5k-70k | x/Σy: 5k-70k |
| x: 6k-300k | x: 6k-300k | x: 6k-300k | x: 6k-300k | x: 6k-300k | x: 6k-300k | x: 6k-300k |
| y: 0.15-2.0 | y: 0.15-2.0 | y: 0.15-2.0 | y: 0.15-2.0 | y: 0.15-2.0 | y: 0.15-2.0 | y: 0.15-2.0 |
| x/Σy: 5k-70k | x/Σy: 5k-70k | x/Σy: 5k-70k | x/Σy: 5k-70k | x/Σy: 5k-70k | x/Σy: 5k-70k | x/Σy: 5k-70k |
| x: 6k-300k | x: 6k-300k | x: 6k-300k | x: 6k-300k | x: 6k-300k | x: 6k-300k | x: 6k-300k |
| y: 0.20-1.5 | y: 0.20-1.5 | y: 0.20-1.5 | y: 0.20-1.5 | y: 0.20-1.5 | y: 0.20-1.5 | y: 0.20-1.5 |
| x/Σy: 5k-70k | x/Σy: 5k-70k | x/Σy: 5k-70k | x/Σy: 5k-70k | x/Σy: 5k-70k | x/Σy: 5k-70k | x/Σy: 5k-70k |
| x: 6k-300k | x: 6k-300k | x: 6k-300k | x: 6k-300k | x: 6k-300k | x: 6k-300k | x: 6k-300k |
| y: 0.20-0.50 | y: 0.20-0.50 | y: 0.20-0.50 | y: 0.20-0.50 | y: 0.20-0.50 | y: 0.20-0.50 | y: 0.20-0.50 |
| x/Σy: 5k-70k | x/Σy: 5k-70k | x/Σy: 5k-70k | x/Σy: 5k-70k | x/Σy: 5k-70k | x/Σy: 5k-70k | x/Σy: 5k-70k |
| x: 9k-35k | x: 9k-35k | x: 9k-35k | x: 9k-35k | x: 9k-35k | x: 9k-35k | x: 9k-35k |
| y: 0.15-2.0 | y: 0.15-2.0 | y: 0.15-2.0 | y: 0.15-2.0 | y: 0.15-2.0 | y: 0.15-2.0 | y: 0.15-2.0 |
| x/Σy: 5k-70k | x/Σy: 5k-70k | x/Σy: 5k-70k | x/Σy: 5k-70k | x/Σy: 5k-70k | x/Σy: 5k-70k | x/Σy: 5k-70k |
| x: 9k-35k | x: 9k-35k | x: 9k-35k | x: 9k-35k | x: 9k-35k | x: 9k-35k | x: 9k-35k |
| y: 0.20-1.5 | y: 0.20-1.5 | y: 0.20-1.5 | y: 0.20-1.5 | y: 0.20-1.5 | y: 0.20-1.5 | y: 0.20-1.5 |
| x/Σy: 5k-70k | x/Σy: 5k-70k | x/Σy: 5k-70k | x/Σy: 5k-70k | x/Σy: 5k-70k | x/Σy: 5k-70k | x/Σy: 5k-70k |
| x: 9k-35k | x: 9k-35k | x: 9k-35k | x: 9k-35k | x: 9k-35k | x: 9k-35k | x: 9k-35k |
| y: 0.20-0.50 | y: 0.20-0.50 | y: 0.20-0.50 | y: 0.20-0.50 | y: 0.20-0.50 | y: 0.20-0.50 | y: 0.20-0.50 |
| x/Σy: 5k-70k | x/Σy: 5k-70k | x/Σy: 5k-70k | x/Σy: 5k-70k | x/Σy: 5k-70k | x/Σy: 5k-70k | x/Σy: 5k-70k |
| x: 3k-600k | x: 3k-600k | x: 3k-600k | x: 3k-600k | x: 3k-600k | x: 3k-600k | x: 3k-600k |
| y: 0.10-4.0 | y: 0.10-4.0 | y: 0.10-4.0 | y: 0.10-4.0 | y: 0.10-4.0 | y: 0.10-4.0 | y: 0.10-4.0 |
| x/Σy: 6k-60k | x/Σy: 6k-60k | x/Σy: 6k-60k | x/Σy: 6k-60k | x/Σy: 6k-60k | x/Σy: 6k-60k | x/Σy: 6k-60k |
| x: 6k-300k | x: 6k-300k | x: 6k-300k | x: 6k-300k | x: 6k-300k | x: 6k-300k | x: 6k-300k |
| y: 0.10-4.0 | y: 0.10-4.0 | y: 0.10-4.0 | y: 0.10-4.0 | y: 0.10-4.0 | y: 0.10-4.0 | y: 0.10-4.0 |
| x/Σy: 6k-60k | x/Σy: 6k-60k | x/Σy: 6k-60k | x/Σy: 6k-60k | x/Σy: 6k-60k | x/Σy: 6k-60k | x/Σy: 6k-60k |
| x: 8k-100k | x: 8k-100k | x: 8k-100k | x: 8k-100k | x: 8k-100k | x: 8k-100k | x: 8k-100k |
| y: 0.10-4.0 | y: 0.10-4.0 | y: 0.10-4.0 | y: 0.10-4.0 | y: 0.10-4.0 | y: 0.10-4.0 | y: 0.10-4.0 |
| x/Σy: 6k-60k | x/Σy: 6k-60k | x/Σy: 6k-60k | x/Σy: 6k-60k | x/Σy: 6k-60k | x/Σy: 6k-60k | x/Σy: 6k-60k |
| x: 9k-35k | x: 9k-35k | x: 9k-35k | x: 9k-35k | x: 9k-35k | x: 9k-35k | x: 9k-35k |
| y: 0.10-4.0 | y: 0.10-4.0 | y: 0.10-4.0 | y: 0.10-4.0 | y: 0.10-4.0 | y: 0.10-4.0 | y: 0.10-4.0 |
| x/Σy: 6k-60k | x/Σy: 6k-60k | x/Σy: 6k-60k | x/Σy: 6k-60k | x/Σy: 6k-60k | x/Σy: 6k-60k | x/Σy: 6k-60k |
| x: 3k-600k | x: 3k-600k | x: 3k-600k | x: 3k-600k | x: 3k-600k | x: 3k-600k | x: 3k-600k |
| y: 0.15-2.0 | y: 0.15-2.0 | y: 0.15-2.0 | y: 0.15-2.0 | y: 0.15-2.0 | y: 0.15-2.0 | y: 0.15-2.0 |
| x/Σy: 6k-60k | x/Σy: 6k-60k | x/Σy: 6k-60k | x/Σy: 6k-60k | x/Σy: 6k-60k | x/Σy: 6k-60k | x/Σy: 6k-60k |
| x: 3k-600k | x: 3k-600k | x: 3k-600k | x: 3k-600k | x: 3k-600k | x: 3k-600k | x: 3k-600k |
| y: 0.20-1.5 | y: 0.20-1.5 | y: 0.20-1.5 | y: 0.20-1.5 | y: 0.20-1.5 | y: 0.20-1.5 | y: 0.20-1.5 |
| x/Σy: 6k-60k | x/Σy: 6k-60k | x/Σy: 6k-60k | x/Σy: 6k-60k | x/Σy: 6k-60k | x/Σy: 6k-60k | x/Σy: 6k-60k |
| x: 3k-600k | x: 3k-600k | x: 3k-600k | x: 3k-600k | x: 3k-600k | x: 3k-600k | x: 3k-600k |
| y: 0.20-0.50 | y: 0.20-0.50 | y: 0.20-0.50 | y: 0.20-0.50 | y: 0.20-0.50 | y: 0.20-0.50 | y: 0.20-0.50 |
| x/Σy: 6k-60k | x/Σy: 6k-60k | x/Σy: 6k-60k | x/Σy: 6k-60k | x/Σy: 6k-60k | x/Σy: 6k-60k | x/Σy: 6k-60k |
| x: 6k-300k | x: 6k-300k | x: 6k-300k | x: 6k-300k | x: 6k-300k | x: 6k-300k | x: 6k-300k |
| y: 0.15-2.0 | y: 0.15-2.0 | y: 0.15-2.0 | y: 0.15-2.0 | y: 0.15-2.0 | y: 0.15-2.0 | y: 0.15-2.0 |
| x/Σy: 6k-60k | x/Σy: 6k-60k | x/Σy: 6k-60k | x/Σy: 6k-60k | x/Σy: 6k-60k | x/Σy: 6k-60k | x/Σy: 6k-60k |
| x: 6k-300k | x: 6k-300k | x: 6k-300k | x: 6k-300k | x: 6k-300k | x: 6k-300k | x: 6k-300k |
| y: 0.20-1.5 | y: 0.20-1.5 | y: 0.20-1.5 | y: 0.20-1.5 | y: 0.20-1.5 | y: 0.20-1.5 | y: 0.20-1.5 |
| x/Σy: 6k-60k | x/Σy: 6k-60k | x/Σy: 6k-60k | x/Σy: 6k-60k | x/Σy: 6k-60k | x/Σy: 6k-60k | x/Σy: 6k-60k |
| x: 6k-300k | x: 6k-300k | x: 6k-300k | x: 6k-300k | x: 6k-300k | x: 6k-300k | x: 6k-300k |
| y: 0.20-0.50 | y: 0.20-0.50 | y: 0.20-0.50 | y: 0.20-0.50 | y: 0.20-0.50 | y: 0.20-0.50 | y: 0.20-0.50 |
| x/Σy: 6k-60k | x/Σy: 6k-60k | x/Σy: 6k-60k | x/Σy: 6k-60k | x/Σy: 6k-60k | x/Σy: 6k-60k | x/Σy: 6k-60k |
| x: 9k-35k | x: 9k-35k | x: 9k-35k | x: 9k-35k | x: 9k-35k | x: 9k-35k | x: 9k-35k |
| y: 0.15-2.0 | y: 0.15-2.0 | y: 0.15-2.0 | y: 0.15-2.0 | y: 0.15-2.0 | y: 0.15-2.0 | y: 0.15-2.0 |
| x/Σy: 6k-60k | x/Σy: 6k-60k | x/Σy: 6k-60k | x/Σy: 6k-60k | x/Σy: 6k-60k | x/Σy: 6k-60k | x/Σy: 6k-60k |
| x: 9k-35k | x: 9k-35k | x: 9k-35k | x: 9k-35k | x: 9k-35k | x: 9k-35k | x: 9k-35k |
| y: 0.20-1.5 | y: 0.20-1.5 | y: 0.20-1.5 | y: 0.20-1.5 | y: 0.20-1.5 | y: 0.20-1.5 | y: 0.20-1.5 |
| x/Σy: 6k-60k | x/Σy: 6k-60k | x/Σy: 6k-60k | x/Σy: 6k-60k | x/Σy: 6k-60k | x/Σy: 6k-60k | x/Σy: 6k-60k |
| x: 9k-35k | x: 9k-35k | x: 9k-35k | x: 9k-35k | x: 9k-35k | x: 9k-35k | x: 9k-35k |
| y: 0.20-0.50 | y: 0.20-0.50 | y: 0.20-0.50 | y: 0.20-0.50 | y: 0.20-0.50 | y: 0.20-0.50 | y: 0.20-0.50 |
| x/Σy: 6k-60k | x/Σy: 6k-60k | x/Σy: 6k-60k | x/Σy: 6k-60k | x/Σy: 6k-60k | x/Σy: 6k-60k | x/Σy: 6k-60k |
| x: 3k-600k | x: 3k-600k | x: 3k-600k | x: 3k-600k | x: 3k-600k | x: 3k-600k | x: 3k-600k |
| y: 0.10-4.0 | y: 0.10-4.0 | y: 0.10-4.0 | y: 0.10-4.0 | y: 0.10-4.0 | y: 0.10-4.0 | y: 0.10-4.0 |
| x/Σy: 6k-60k | x/Σy: 6k-60k | x/Σy: 6k-60k | x/Σy: 6k-60k | x/Σy: 6k-60k | x/Σy: 6k-60k | x/Σy: 6k-60k |
| x: 6k-300k | x: 6k-300k | x: 6k-300k | x: 6k-300k | x: 6k-300k | x: 6k-300k | x: 6k-300k |
| y: 0.10-4.0 | y: 0.10-4.0 | y: 0.10-4.0 | y: 0.10-4.0 | y: 0.10-4.0 | y: 0.10-4.0 | y: 0.10-4.0 |
| x/Σy: 6k-60k | x/Σy: 6k-60k | x/Σy: 6k-60k | x/Σy: 6k-60k | x/Σy: 6k-60k | x/Σy: 6k-60k | x/Σy: 6k-60k |
| x: 8k-100k | x: 8k-100k | x: 8k-100k | x: 8k-100k | x: 8k-100k | x: 8k-100k | x: 8k-100k |
| y: 0.10-4.0 | y: 0.10-4.0 | y: 0.10-4.0 | y: 0.10-4.0 | y: 0.10-4.0 | y: 0.10-4.0 | y: 0.10-4.0 |
| x/Σy: 6k-60k | x/Σy: 6k-60k | x/Σy: 6k-60k | x/Σy: 6k-60k | x/Σy: 6k-60k | x/Σy: 6k-60k | x/Σy: 6k-60k |
| x: 9k-35k | x: 9k-35k | x: 9k-35k | x: 9k-35k | x: 9k-35k | x: 9k-35k | x: 9k-35k |
| y: 0.10-4.0 | y: 0.10-4.0 | y: 0.10-4.0 | y: 0.10-4.0 | y: 0.10-4.0 | y: 0.10-4.0 | y: 0.10-4.0 |
| x/Σy: 6k-60k | x/Σy: 6k-60k | x/Σy: 6k-60k | x/Σy: 6k-60k | x/Σy: 6k-60k | x/Σy: 6k-60k | x/Σy: 6k-60k |
| x: 3k-600k | x: 3k-600k | x: 3k-600k | x: 3k-600k | x: 3k-600k | x: 3k-600k | x: 3k-600k |
| y: 0.10-4.0 | y: 0.10-4.0 | y: 0.10-4.0 | y: 0.10-4.0 | y: 0.10-4.0 | y: 0.10-4.0 | y: 0.10-4.0 |
| x/Σy: 7k-50k | x/Σy: 7k-50k | x/Σy: 7k-50k | x/Σy: 7k-50k | x/Σy: 7k-50k | x/Σy: 7k-50k | x/Σy: 7k-50k |
| x: 6k-300k | x: 6k-300k | x: 6k-300k | x: 6k-300k | x: 6k-300k | x: 6k-300k | x: 6k-300k |
| y: 0.10-4.0 | y: 0.10-4.0 | y: 0.10-4.0 | y: 0.10-4.0 | y: 0.10-4.0 | y: 0.10-4.0 | y: 0.10-4.0 |
| x/Σy: 7k-50k | x/Σy: 7k-50k | x/Σy: 7k-50k | x/Σy: 7k-50k | x/Σy: 7k-50k | x/Σy: 7k-50k | x/Σy: 7k-50k |
| x: 8k-100k | x: 8k-100k | x: 8k-100k | x: 8k-100k | x: 8k-100k | x: 8k-100k | x: 8k-100k |
| y: 0.10-4.0 | y: 0.10-4.0 | y: 0.10-4.0 | y: 0.10-4.0 | y: 0.10-4.0 | y: 0.10-4.0 | y: 0.10-4.0 |
| x/Σy: 7k-50k | x/Σy: 7k-50k | x/Σy: 7k-50k | x/Σy: 7k-50k | x/Σy: 7k-50k | x/Σy: 7k-50k | x/Σy: 7k-50k |
| x: 9k-35k | x: 9k-35k | x: 9k-35k | x: 9k-35k | x: 9k-35k | x: 9k-35k | x: 9k-35k |
| y: 0.10-4.0 | y: 0.10-4.0 | y: 0.10-4.0 | y: 0.10-4.0 | y: 0.10-4.0 | y: 0.10-4.0 | y: 0.10-4.0 |
| x/Σy: 7k-50k | x/Σy: 7k-50k | x/Σy: 7k-50k | x/Σy: 7k-50k | x/Σy: 7k-50k | x/Σy: 7k-50k | x/Σy: 7k-50k |
| x: 3k-600k | x: 3k-600k | x: 3k-600k | x: 3k-600k | x: 3k-600k | x: 3k-600k | x: 3k-600k |
| y: 0.15-2.0 | y: 0.15-2.0 | y: 0.15-2.0 | y: 0.15-2.0 | y: 0.15-2.0 | y: 0.15-2.0 | y: 0.15-2.0 |
| x/Σy: 7k-50k | x/Σy: 7k-50k | x/Σy: 7k-50k | x/Σy: 7k-50k | x/Σy: 7k-50k | x/Σy: 7k-50k | x/Σy: 7k-50k |
| x: 3k-600k | x: 3k-600k | x: 3k-600k | x: 3k-600k | x: 3k-600k | x: 3k-600k | x: 3k-600k |
| y: 0.20-1.5 | y: 0.20-1.5 | y: 0.20-1.5 | y: 0.20-1.5 | y: 0.20-1.5 | y: 0.20-1.5 | y: 0.20-1.5 |
| x/Σy: 7k-50k | x/Σy: 7k-50k | x/Σy: 7k-50k | x/Σy: 7k-50k | x/Σy: 7k-50k | x/Σy: 7k-50k | x/Σy: 7k-50k |
| x: 3k-600k | x: 3k-600k | x: 3k-600k | x: 3k-600k | x: 3k-600k | x: 3k-600k | x: 3k-600k |
| y: 0.20-0.50 | y: 0.20-0.50 | y: 0.20-0.50 | y: 0.20-0.50 | y: 0.20-0.50 | y: 0.20-0.50 | y: 0.20-0.50 |
| x/Σy: 7k-50k | x/Σy: 7k-50k | x/Σy: 7k-50k | x/Σy: 7k-50k | x/Σy: 7k-50k | x/Σy: 7k-50k | x/Σy: 7k-50k |
| x: 6k-300k | x: 6k-300k | x: 6k-300k | x: 6k-300k | x: 6k-300k | x: 6k-300k | x: 6k-300k |
| y: 0.15-2.0 | y: 0.15-2.0 | y: 0.15-2.0 | y: 0.15-2.0 | y: 0.15-2.0 | y: 0.15-2.0 | y: 0.15-2.0 |
| x/Σy: 7k-50k | x/Σy: 7k-50k | x/Σy: 7k-50k | x/Σy: 7k-50k | x/Σy: 7k-50k | x/Σy: 7k-50k | x/Σy: 7k-50k |
| x: 6k-300k | x: 6k-300k | x: 6k-300k | x: 6k-300k | x: 6k-300k | x: 6k-300k | x: 6k-300k |
| y: 0.20-1.5 | y: 0.20-1.5 | y: 0.20-1.5 | y: 0.20-1.5 | y: 0.20-1.5 | y: 0.20-1.5 | y: 0.20-1.5 |
| x/Σy: 7k-50k | x/Σy: 7k-50k | x/Σy: 7k-50k | x/Σy: 7k-50k | x/Σy: 7k-50k | x/Σy: 7k-50k | x/Σy: 7k-50k |
| x: 6k-300k | x: 6k-300k | x: 6k-300k | x: 6k-300k | x: 6k-300k | x: 6k-300k | x: 6k-300k |
| y: 0.20-0.50 | y: 0.20-0.50 | y: 0.20-0.50 | y: 0.20-0.50 | y: 0.20-0.50 | y: 0.20-0.50 | y: 0.20-0.50 |
| x/Σy: 7k-50k | x/Σy: 7k-50k | x/Σy: 7k-50k | x/Σy: 7k-50k | x/Σy: 7k-50k | x/Σy: 7k-50k | x/Σy: 7k-50k |
| x: 9k-35k | x: 9k-35k | x: 9k-35k | x: 9k-35k | x: 9k-35k | x: 9k-35k | x: 9k-35k |
| y: 0.15-2.0 | y: 0.15-2.0 | y: 0.15-2.0 | y: 0.15-2.0 | y: 0.15-2.0 | y: 0.15-2.0 | y: 0.15-2.0 |
| x/Σy: 7k-50k | x/Σy: 7k-50k | x/Σy: 7k-50k | x/Σy: 7k-50k | x/Σy: 7k-50k | x/Σy: 7k-50k | x/Σy: 7k-50k |
| x: 9k-35k | x: 9k-35k | x: 9k-35k | x: 9k-35k | x: 9k-35k | x: 9k-35k | x: 9k-35k |
| y: 0.20-1.5 | y: 0.20-1.5 | y: 0.20-1.5 | y: 0.20-1.5 | y: 0.20-1.5 | y: 0.20-1.5 | y: 0.20-1.5 |
| x/Σy: 7k-50k | x/Σy: 7k-50k | x/Σy: 7k-50k | x/Σy: 7k-50k | x/Σy: 7k-50k | x/Σy: 7k-50k | x/Σy: 7k-50k |
| x: 9k-35k | x: 9k-35k | x: 9k-35k | x: 9k-35k | x: 9k-35k | x: 9k-35k | x: 9k-35k |
| y: 0.20-0.50 | y: 0.20-0.50 | y: 0.20-0.50 | y: 0.20-0.50 | y: 0.20-0.50 | y: 0.20-0.50 | y: 0.20-0.50 |
| x/Σy: 7k-50k | x/Σy: 7k-50k | x/Σy: 7k-50k | x/Σy: 7k-50k | x/Σy: 7k-50k | x/Σy: 7k-50k | x/Σy: 7k-50k |
| x: 3k-600k | x: 3k-600k | x: 3k-600k | x: 3k-600k | x: 3k-600k | x: 3k-600k | x: 3k-600k |
| y: 0.10-4.0 | y: 0.10-4.0 | y: 0.10-4.0 | y: 0.10-4.0 | y: 0.10-4.0 | y: 0.10-4.0 | y: 0.10-4.0 |
| x/Σy: 7k-50k | x/Σy: 7k-50k | x/Σy: 7k-50k | x/Σy: 7k-50k | x/Σy: 7k-50k | x/Σy: 7k-50k | x/Σy: 7k-50k |
| x: 6k-300k | x: 6k-300k | x: 6k-300k | x: 6k-300k | x: 6k-300k | x: 6k-300k | x: 6k-300k |
| y: 0.10-4.0 | y: 0.10-4.0 | y: 0.10-4.0 | y: 0.10-4.0 | y: 0.10-4.0 | y: 0.10-4.0 | y: 0.10-4.0 |
| x/Σy: 7k-50k | x/Σy: 7k-50k | x/Σy: 7k-50k | x/Σy: 7k-50k | x/Σy: 7k-50k | x/Σy: 7k-50k | x/Σy: 7k-50k |
| x: 8k-100k | x: 8k-100k | x: 8k-100k | x: 8k-100k | x: 8k-100k | x: 8k-100k | x: 8k-100k |
| y: 0.10-4.0 | y: 0.10-4.0 | y: 0.10-4.0 | y: 0.10-4.0 | y: 0.10-4.0 | y: 0.10-4.0 | y: 0.10-4.0 |
| x/Σy: 7k-50k | x/Σy: 7k-50k | x/Σy: 7k-50k | x/Σy: 7k-50k | x/Σy: 7k-50k | x/Σy: 7k-50k | x/Σy: 7k-50k |
| x: 9k-35k | x: 9k-35k | x: 9k-35k | x: 9k-35k | x: 9k-35k | x: 9k-35k | x: 9k-35k |
| y: 0.10-4.0 | y: 0.10-4.0 | y: 0.10-4.0 | y: 0.10-4.0 | y: 0.10-4.0 | y: 0.10-4.0 | y: 0.10-4.0 |
| x/Σy: 7k-50k | x/Σy: 7k-50k | x/Σy: 7k-50k | x/Σy: 7k-50k | x/Σy: 7k-50k | x/Σy: 7k-50k | x/Σy: 7k-50k |

| | | | | | | |
|---|---|---|---|---|---|---|
| k= ^{∗}10³ | | | | | | |

The skilled person will understand that under force the volume of a body can change. This is in particular the case for bodies made of pliable material, such as pliable film. In case of the use of pliable materials the volume of the mite compartment may vary between the volume of the material present in the mite compartment (e.g. the mite composition comprising the individuals of the mite population and often a carrier) and the maximal volume that the material enclosing the mite compartment, the gas barrier material, may provide on the basis of its dimensions and/or geometrical restrictions. Thus for mite releasing systems using pliable gas barrier material, the value x, may not be fixed but may vary. For such systems the relevant volume of the mite compartment to take into consideration for determining the xΣy ratio is the volume the mite compartment has during a substantial amount of time, such as during at least 12 hours, such as at least 18 hours.

The barrier material used preferably is opaque, thus preventing light to enter the mite compartment. This is beneficial to prevent heat absorption from visible light in the mite compartment. The NatureFlex^{™}N932 (Innovia^{™} Films) film and the BUI43 foil (obtainable from Euroflex B.V., Zwolle, The Netherlands) are examples of gas barriers having opaque properties.

In view of sustainable use of the system of the invention, it is further preferred that the system is made from compostable materials. The use of compostable gas barrier materials in this respect is preferred. The NatureFlex^{™} N932 (Innovia^{™} Films) film and the BUI43 foil (obtainable from Euroflex B.V., Zwolle, The Netherlands) are examples of compostable gas barrier materials having suitable properties.

Further aspects of the invention relates to the use of the system according to the invention for introducing beneficial mites in a target area. The target area may be any area where the activity of the beneficial mites is desired. The beneficial mites may be predatory mites or mites suitable as a food source for predatory mites or for other predatory beneficial arthropods. As will be clear from the present description, in case the beneficial mites are selected from a predatory mite species, a mite species suitable as a food source for the predatory mites may also be present in the mite compartment of the system according to the invention. As will also be clear from the present description, in case the beneficial mites are selected from a mite species suitable as a food source for predatory mites or for other predatory arthropods, the predatory mites preferably are not present in the mite compartment of the system according to the invention. Or described differently, according to such embodiments, the population of beneficial mites preferably consists of a number of mite species suitable as a food source for predatory mites or for other predatory arthropods. For example in case the beneficial mites are predatory mites having a function in controlling crop pests, the target area may be a crop. The crop may be selected from, but are not restricted to (greenhouse) vegetable crops such as tomatoes (*Solanum lycopersicum*), peppers (*Capsicum annuum*)*,* eggplants (*Solanum melogena*) Curcubits (*Cucurbitaceae*) such as cucumbers (*cucumis sativa*), melons (*cucumis melo*) watermelons (*Citrullus lanatus*); soft fruit (such as strawberries (*Fragaria x annanassa*), raspberries (*Rubus ideaus*)), blueberries, (greenhouse) ornamental crops (such as roses, gerberas, chrysanthemums) or tree crops such as *Citrus spp.* Mites suitable as a food source for predatory mites or for other predatory arthropods having a function in controlling crop pests may also be released in a crop in order to support the population development of predatory species present in the crop. The predatory mite may be a Mesostigmatid or Prostigmatid species as presented above. Other predatory arthropods may be selected form the family Miridae, such as Macrolophus spp., from the family Anthocoridae, such as Orius spp., for example *Orius laevigatus,* from the family Coccinellidae, such as [Adalia spp. or *Cryptolaemus montrouzieri,* from the Chrysopidae, such as Chrysoperla spp., for example *Chrysoperla carnea.*

According to alternative embodiments, the beneficial mites may have a function in controlling pests of an animal, the host animal, in particular pests of domestic animals, including farm animals and companion animals, such as poultry, cattle, horses, dogs or cats. According to such embodiments the target area may be a stable or sleeping area for the host animal. The system according to the invention may for example be used in support of the control of poultry red mite, by comprising as the beneficial mite a predatory mites selected from the genus *Hypoaspis,* such as *Hypoaspis angusta,* from the genus *Cheyletus,* such as *Cheyletus eruditis,* from the genus *Androlaelaps,* such as *Androlaelaps casalis,* from the family *Laelapidae* such as from the genus *Stratiolaelaps* e.g. *Stratiolaelaps scimitus* (Womersley); *Gaeolaelaps* e.g. *Gaeolaelaps aculeifer* (Canestrini); *Androlaelaps* e.g. *Androlaelaps casalis* (Berlese), or from the genus *Macrocheles,* such as *Macrocheles robustulus* or an Astigmatid mite suitable as prey for a predatory mite from this selection. As the skilled person knows, these predatory mites have broader host ranges and thus may also be employed for controlling other pests. In addition other beneficial predatory arthropods may also be used to control pests of animal hosts. The system of the invention may be used to release Astigmatid mites that may serve as a food source for such beneficial predatory arthropods and thus may support the survival and/or development of their populations, thus supporting the control of the pest of the animal host.

In yet other embodiments the beneficial mites are predators for pests of stored food products, such as stored product mites. In such embodiments the target area is a food product storage.

In the use of the invention the beneficial mite is introduced in the target area, by providing the system of the invention in the target area or in the proximity thereof. This may be done by placing the system of the invention in the target area or hanging it in the target area. For hanging in the target area, the system of the invention according to certain embodiments may comprise hanging means, such as a number of hooks and or a number of threads. Such hanging means may be fixed to gas barrier material.

As is shown in the experiments below, the mite releasing system according to the invention maintains adequate functions when used in an environment wherein the ambient relative humidity (RH) is below 70%. This provides a system that is more robust and that may be employed under conditions where the RH fluctuates to values below 70% or even on average is below 70%. In view of the fact that environmental conditions may not always be controllable, the present invention provides a system with a reduced risk of failure due to too low ambient humidity. Therefore, according to certain preferred embodiments the system of the invention is for use in an environment wherein the ambient relative humidity (RH) may reach values below 65%, such as 65% - 10%, or below 60%, below 55%, below 50%, below 45%, below 40%, below 30%, below 25%, below 20%, or below 15%. According to other preferred embodiments, the system of the invention is for use in an environment wherein the average ambient relative humidity (RH) is below 65%, such as 65% - 10%, or below 60%, below 55%, below 50%, below 45%, below 40%, below 30%, below 25%, below 20%, or below 15%.

A further aspect of the invention relates to a method for controlling a pest susceptible of being preyed by a predatory mite species or other beneficial predatory arthropod species comprising providing the system according to the invention to a target area where the pest is to be controlled.

Yet a further aspect of the invention relates to a method for producing an agricultural product from a number of non-human organisms prone to infestation by a pest susceptible of being preyed by a predatory beneficial arthropod, said method comprising:
- providing the number of non-human organisms in an area, the target area;
- providing in or in the proximity of the target area a number of systems according to the invention;
- providing to the number of non-human organisms suitable nutrients and environmental conditions to produce the agricultural product.

The number of non-human organisms may be selected from a crop species (as defined previously), an avian species, preferably a poultry species, such as chickens or turkeys, mammalian livestock.

A pest susceptible of being preyed by a predatory mite species should be understood as referring to a pest that is a suitable prey for a predatory mite present in the mite releasing system (the predatory mite selected as the beneficial mite).

A non-human organisms prone to infestation by a pest susceptible of being preyed by a predatory mite species should be understood as referring to a non-human organism that is prone to attract a pest, said pest being a suitable prey for a predatory mite present in the mite releasing system (the predatory mite selected as the beneficial mite). The non-human organisms prone to infestation by a pest thus is a suitable host for the pest and the pest is a suitable prey for the predatory mite present in the mite releasing system (the predatory mite selected as the beneficial mite).

Agricultural products that may be produced from a crop may include any plant material having agricultural value, such as plant biomass, seeds, fruits etcetera. Agricultural products that may be produced from an avian species such as poultry, in particular chickens or turkeys may include meat, eggs and manure. Agricultural products that may be produced from mammalian livestock, such as cattle, goats, sheep, pigs, may include meat and leather and manure.

The various embodiments of this aspect of the invention and the technical details connected thereto are similar to those of the use of the system for introducing beneficial mites in a target area as discussed above.

The invention will now be further illustrated with reference to the attached figures and the example presented below. It should be emphasized that these figures, the description relating thereto and the example are only illustrative and by no means restrict the scope of the invention as defined in the claims.

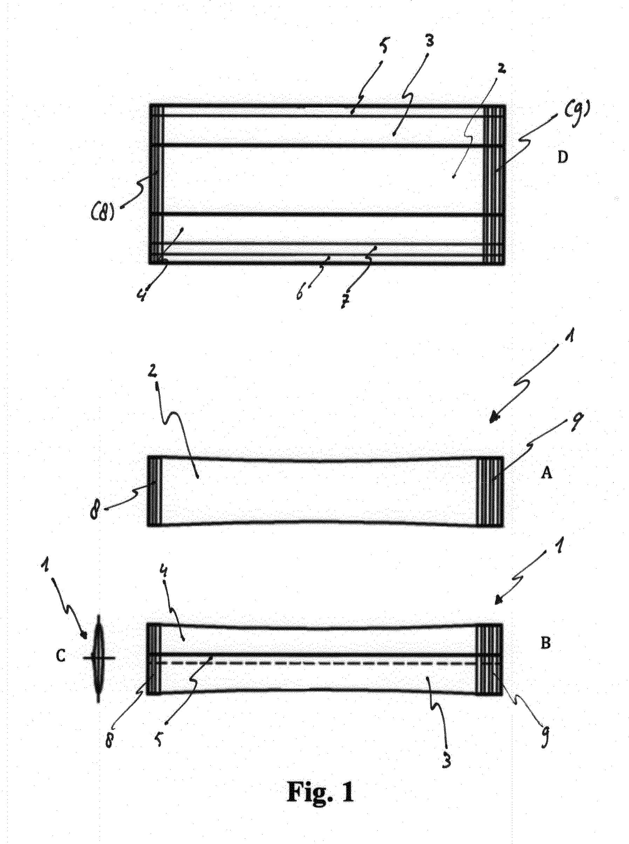

Figure 1 schematically shows a mite releasing system (1) according to an embodiment of the invention having the form of a stick shaped sachet. Figure 1A presents a view on the front side of the mite releasing system (1) where the frontal panel (2) is located. figure 1B presents a view on the rear side of the mite releasing system (1) where a first rear panel (3) and a second rear panel (4) and the back of the sealing surface (5) are located. Figure 1C presents a view in the direction of the longest axis of the elongated mite releasing system (1). The stick shaped sachet (1) is folded from a planar foil (BUI 43, Euroflex B.V., Zwolle, The Netherlands) shown in figure 1D with the exterior side facing upward. The parts forming the frontal panel (2) (35 mm wide and 85 mm long), the first rear panel (3), the second rear panel (4) and the sealing fin (5) in the folded conformation of the mite releasing system (1) are indicated. In addition in figure 1D a second sealing surface (6) that joins with sealing surface 5 and fold (7) are presented. In the folded and sealed conformation the fold (7) and the second seal surface (6) covered by seal surface (5) are not visible. The folded configuration presented in figures 1A, 1B and 1C is obtained in a procedure similar to the procedures for producing sugar sticks and coffee creamer sticks using similar machines. For this sealing surface (5) is joined with sealing surface (6) and the parts are sealed at a suitable temperature above the sealing temperature of the material. A fold is then created along the line between parts (6) and (7) to allow the seal fin to bend back to the body of the stick. This allows the seal fin to be attached to the body of the stick on the second rear panel (4). Next the lower seal (8) is executed. This creates an open container that is filled with a mite composition comprising a mite population on a carrier. After filling, the upper seal (9) is executed. This upper seal (9) is broader than lower seal (8) in order to provide an attachment point for a hanging means, such as a cardboard hook (not shown). In figure 1D the locations of the lower seal (8) and upper seal (9) are presented with reference numbers in brackets, in view of the fact that in the planar unfolded situation the seals are not actually present.

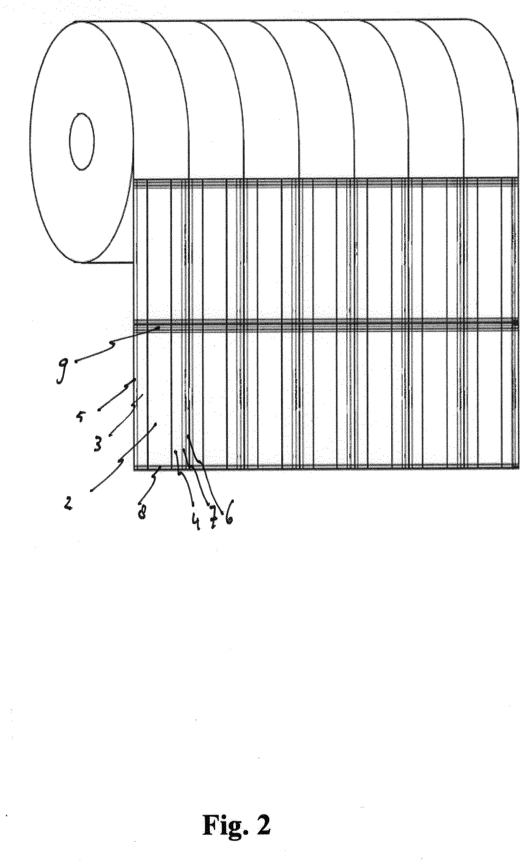

Figure 2 shows how multiple mite releasing sachets can be formed from a roll of foil. For a single planar piece of foil the parts forming the frontal panel (2), the first rear panel (3), the second rear panel (4) and the sealing fin (5) in the folded conformation of the mite releasing system are indicated. In addition inside fin flap (7), part (6) covered by the fin seal and the parts where heat seals (8) and (9) will be positioned are indicated. Cutting, folding, sealing, filling with a mite composition comprising a mite population in association with a carrier, and introduction of the opening (10) to connect the mite compartment with the space outside the mite compartment may be performed fully automated with technology and procedures similar to the technology and procedures used for producing sugar sticks and coffee creamer sticks.

### EXAMPLE

### Mite cultures

A stock rearing of *Amblyseius swirskii* on the prey mite *Carpoglyphus lactis* on a carrier material of humidified bran (20 % w/w water content). Nutrients for C. *lactis* were provided by the farinaceous material of the bran and 5 % (w/w) yeast extract added to the bran. The number of mites in the rearing mixture was assessed using standard counting methods as disclosed in van Lenteren, J.C., Hale, A., Klapwijk, J.N., van Schelt, J. and S. Steinberg (2003) Guidelines for quality control of commercially produced natural enemies. In: van Lenteren, J.C. (ed) Quality control and production of biological control agents: Theory and testing procedures CABI Publishing, Wallingford UK, pp 293-294.

### Procedure

Mite releasing systems (sachets) having the following design variations of the mite compartment were compared:
1. Polyethylene (PE) coated paper (Kraft paper 40g/m² laminated with extruded PE 17 g/m² (KBM 40+17gr) Burgo, Italy), standard^{∗} form of the mite compartment and a single opening with a diameter of 0.65 ±0.05 mm connecting to the space outside the mite compartment.
2. PE coated paper (Kraft paper 40g/m² laminated with extruded PE 17 g/m² (KBM 40+17gr) Burgo, Italy), standard^{∗} form of the mite compartment and a single opening with a diameter of 1.3 mm connecting to the space outside the mite compartment.
3. BUI43 foil (Euroflex B.V., Zwolle, The Netherlands), standard^{∗} form of the mite compartment and a single opening with a diameter of 0.65 ± 0.05 mm connecting to the space outside the mite compartment.
4. BUI 43 foil (Euroflex B.V., Zwolle, The Netherlands), standard^{∗} form of the mite compartment and a single opening with a diameter of 1.3 mm connecting to the space outside the mite compartment.
5. BUI 43 foil (Euroflex B.V., Zwolle, The Netherlands), stick^{∗∗} form (stick shape) of the mite compartment and a single opening with a diameter of 0.65 ± 0.05 mm connecting to the space outside the mite compartment.

^{∗}Standard form is as used in standard mite release system (sachet) of Koppert Biological Systems (Berkel en Rodenrijs, the Netherlands) used at present in the SWIRSKI-MITE PLUS, products (mite compartment size excluding the seal strips: 50 x 50 mm). On the basis of these dimensions, the volume of the material filled (2,3 grams of a carrier material corresponding to about 11,5 cc) and the head space maintained, the volume of the interior of the mite compartment (x) was determined to be about 14 cc.

^{∗∗}Stick form is alternative shape according to certain embodiments of the invention (mite compartment size excluding the seal strips: 35 x 65 mm). On the basis of these dimensions, the volume of the material filled (2,3 grams of a carrier material corresponding to about 11,5 cc) and the head space maintained, the volume of the interior of the mite compartment (x) was determined to be about 14 cc.

The BUI sachets were made manually with a hand-sealing machine and the PE paper sachets were produced in the production facilities of Koppert B.V. according to the specifications for the SWIRSKI-MITE PLUS product. Near the top end of the sachets a single opening with the diameter of 0.65 ±0.05 mm (y = π^{∗}(0.65/2)² = 0.33 mm²) or with a diameter of 1.3 mm (y = π^{∗}(1.3/2)² = 1.3 mm²) was made with two different types of needles having shafts with diameters of the indicated sizes. Both the 0.65 and the 1.3 mm diameter opening are relatively small in respect of what is used in the prior art.

Mite countings according to standard methods (van Lenteren et al., 2003 supra) carried out on the carrier material of humidified bran and nutrients revealed that it contained approx. 112 A. *swirskii* and 277 C. *lactis* per gram at the beginning of the experiment. 2.3 grams (about 11,5 cc) of the carrier material were filled into the sachets (resulting in approx. 257 A. *swirskii* and approx. 637 C. *lactis* per sachet). Thereafter the sachets were sealed. In this way 45 sachets of each type were prepared.

36 sachets of each type were hung alternately on a cotton thread using paperclips in a climate cabinet regulated at 22 degrees Celsius and a relative humidity of 50 %. Twice a week, 3 sachets of each type were sampled in the following manner. The sachets were opened and the content of the 3 sachets of the same type was mixed and the number of mites in the mixture was assessed using standard counting methods (van Lenteren et al., 2003 supra). At the same time the water activity (Rotronic HP23-AW-A with HC2-AW) and the moisture content (Sartorius MA150) of the carrier material were measured. This procedure was repeated until the number of mites in the sachets decreased significantly.

At the same time the other sachets were used for a walking out test.

From each type, 3 sachets were placed together into a glass jar. Each glass jar was placed separately in a plastic bucket (10 liters) in a layer (2 cm deep) of water to which some drops of soap was added. Buckets were placed into another climate cabinet also regulated at 22 degrees Celsius and a relative humidity of 50 %. Mites (predatory mites an prey mites) escaping the jars we captured in the soapy water solution. Twice a week all glass jars were transferred to new, clean, plastic buckets with new soapy water solution. This procedure was repeated until escape (production) of mites decreased significantly. The mites in the soapy water solution were counted.

### Results

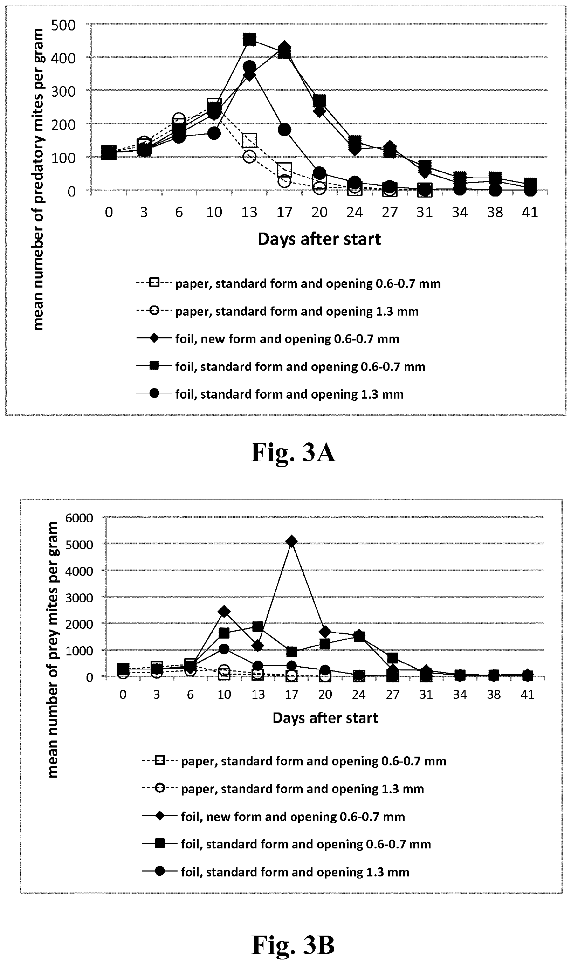

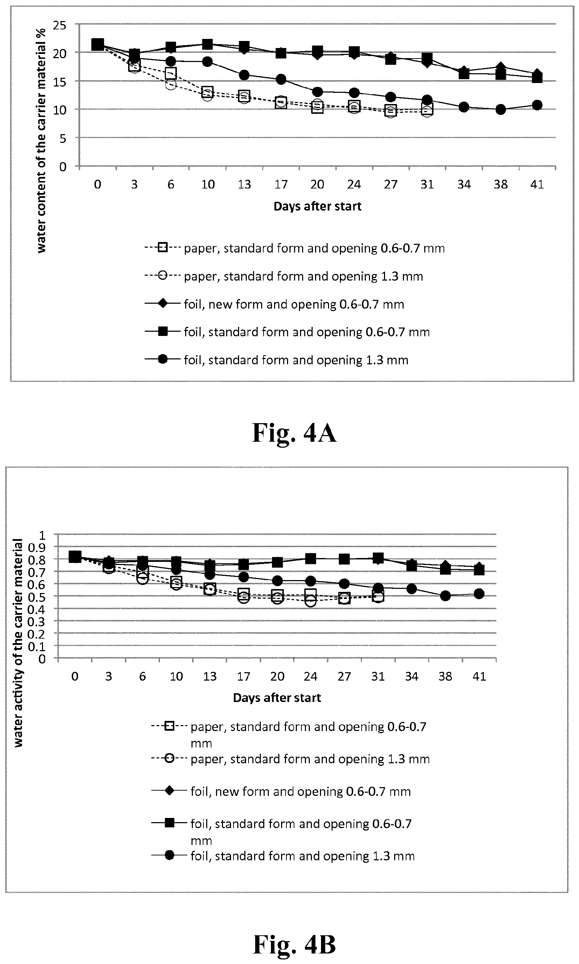

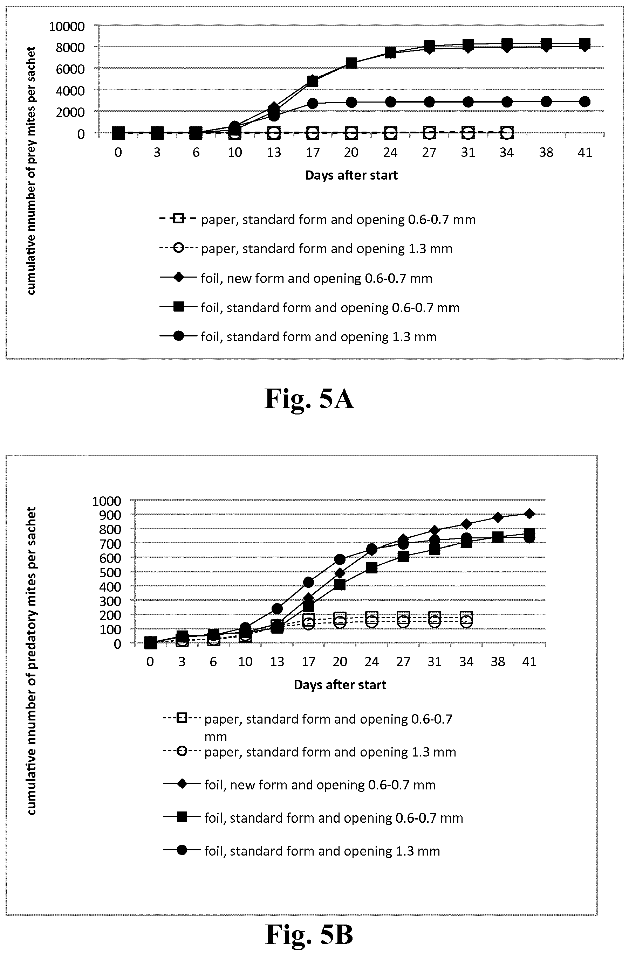

The results of countings of predatory mites (A. *swirskii*) and prey mites (C. *lactis)* inside the mite releasing systems having the different design variations are shown in figures 3A and 3B. Figures 4A and 4B show the values of the water activity (a_{w}) and moisture content over time inside the mite releasing systems having the different design variations. Figures 5A and 5B show the results of countings of predatory mites (A. *swirskii*) and prey mites (C. *lactis)* collected in the soapy water used in the walking out test. These numbers represent the number of mites that actively dispersed out the mite releasing systems during the experiment.

### Conclusions

On the basis of the data presented it can be surprisingly concluded that mite populations can be maintained over prolonged periods in mite releasing systems constructed from materials having a low water vapor transmission rate (and an associated low transfer rate for one or more metabolic gasses) while having only a small opening for gas exchange. More surprisingly, mite population development inside such systems is improved under conditions of 50% RH in comparison to prior art mite releasing systems. Such conditions and lower RH conditions are often encountered in many agricultural settings, in particular in growing outdoor crops (there is at least a risk of these conditions occurring). Thus mite releasing systems according to the invention are better adapted to variations in humidity conditions then prior art mite releasing systems and therefore may be used with less risk of failure in situations where there is a risk of low RH conditions (below 65% or lower, such as below 55%). Furthermore, it is also surprising that mite dispersal out of the mite releasing systems is increased with a decreasing size of the opening connecting the mite compartment and the space outside the mite compartment.

## Claims

1. System for releasing beneficial mites comprising a compartment, the mite compartment, holding a population of a beneficial mite species, preferably in association with a carrier, and a food source for the beneficial mites wherein said mite compartment is enclosed by material, gas barrier material, having a water vapour transmission rate of ≤ 5 g/m^{2∗}24 hours, said mite compartment having a volume of x mm³, wherein x is between 3^{∗}10³ to 600^{∗}10³ mm³ and wherein the system further comprises a number of connections that connect the mite compartment with the space outside the mite compartment, said number of connections each having an area y, wherein y is between 0.1 and 4.0 mm², wherein the sum of the areas of the number of connections is Σy and wherein 5^{∗}10³ mm ≤ x/Σy ≤70^{∗}10³ mm, preferably 6^{∗}10³ mm ≤x/Σy ≤ 60^{∗}10³ mm, more preferably 7^{∗}10³ mm ≤ x/Σy ≤ 50^{∗}10³ mm.

2. System according to claim 1, wherein the beneficial mite species is selected from a predatory mite species, such as a predatory mite species selected from:
- Mesostigmatid mite species such as selected from:
i) *Phytoseiidae* such as from:
- the subfamily of the *Amblyseiinae,* such as from the genus *Amblyseius,* e.g. *Amblyseius andersoni, Amblyseius aerialis, Amblyseius swirskii, Amblyseius herbicolus or Amblyseius largoensis,* from the genus *Euseius* e.g. *Euseius finlandicus, Euseius hibisci, Euseius ovalis, Euseius victoriensis, Euseius stipulatus, Euseius scutalis, Euseius tularensis, Euseius addoensis, Euseius concordis, Euseius ho, Euseius gallicus, Euseius citrifolius* or *Euseius citri,* from the genus *Iphiseiodes* e.g. *Iphiseiodes zuluagi,* from the genus *Iphiseius* e.g. *Iphiseius degenerans,* from the genus *Neoseiulus* e.g. *Neoseiulus barkeri, Neoseiulus californicus, Neoseiulus cucumeris, Neoseiulus longispinosus, Neoseiulus womersleyi, Neoseiulus idaeus, Neoseiulus anonymus, Neoseiulus paspalivorus, Neoseiulus reductus* or *Neoseiulus fallacis, Neoseiulus baraki,* from the genus *Amblydromalus e.g. Amblydromalus limonicus* from the genus *Typhlodromalus* e.g. *Typhlodromalus aripo, Typhlodromalus lailae* or *Typhlodromalus peregrinus* from the genus *Transeius* e.g. *Transeius montdorensis,* from the genus *Phytoseiulus,* e.g. *Phytoseiulus persimilis, Phytoseiulus macropilis, Phytoseiulus longipes, Phytoseiulus fragariae;*
- the subfamily of the *Typhlodrominae,* such as from the genus *Galendromus* e.g. *Galendromus occidentalis,* from the genus *Metaseiulus* e.g. *Metaseiulus flumenis,* from the genus *Gynaeseiu* e.g. *Gynaeseius liturivorus* from the genus *Typhlodromus* e.g. *Typhlodromus exhilarates, Typhlodromus phialatus, Typhlodromus recki, Typhlodromus transvaalensis, Typhlodromus pyri, Typhlodromus doreenae* or *Typhlodromus athiasae;*
ii) *Ascidae* such as from the genus *Proctolaelaps,* such as *Proctolaelaps pygmaeus* (Muller), from the genus *Blattisocius* e.g. *Blattisocius tarsalis* (Berlese), *Blattisocius keegani* (Fox), from the genus *Lasioseius* e.g. *Lasioseius fimetorum* Karg, *Lasioseius floridensis* Berlese, *Lasioseius bispinosus* Evans, *Lasioseius dentatus* Fox, *Lasioseius scapulatus* (Kenett), *Lasioseius athiasae* Nawar & Nasr, from the genus *Arctoseius* e.g. *Arctoseius semiscissus* (Berlese), from the genus *Protogamasellus* e.g. *Protogamasellus dioscorus* Manson;
iii) *Laelapidae* such as from the genus *Stratiolaelaps* e.g. *Stratiolaelaps scimitus* (Womersley), from the genus *Gaeolaelaps* e.g. *Gaeolaelaps aculeifer* (Canestrini), from the genus *Androlaelaps* e.g. *Androlaelaps casalis* (Berlese), from the genus *Cosmolaelaps* e.g. *Cosmolaelaps claviger, Cosmolaelaps jaboticabalensis;*
iv) *Macrochelidae* such as from the genus *Macrocheles* e.g. *Macrocheles robustulus* (Berlese), *Macrocheles muscaedomesticae* (Scopoli), *Macrocheles matrius* (Hull);
v) *Parasitidae* such as from the genus *Pergamasus* e.g. *Pergamasus quisquiliarum* Canestrini, from the genus *Parasitus* e.g. *Parasitus fimetorum* (Berlese), *Parasitus bituberosus, Parasitus mycophilus, Parasitus mammilatus;*
- *Prostigmatid* mite species such as from:
vi) *Tydeidae* such as from the genus *Homeopronematus* e.g. *Homeopronematus anconai* (Baker), from the genus *Tydeus e.g.Tydeus lambi* (Baker), *Tydeus caudatus* (Dugés), from the genus *Pronematus* e.g. *Pronematus ubiquitous* (McGregor);
vii) *Cheyletidae* such as from the genus *Cheyletus* e.g. *Cheyletus eruditus* (Schrank), *Cheyletus malaccensis* Oudemans;
viii) *Cunaxidae* such as from the genus *Coleoscirus e.g.Coleoscirus simplex* (Ewing), from the genus *Cunaxa* e.g. *Cunaxa setirostris* (Hermann);
ix) *Erythraeidae* such as from the genus *Balaustium* e.g. *Balaustium putmani* Smiley, *Balaustium medicagoense* Meyer &Ryke, *Balaustium murorum* (Hermann), *Balaustium hernandezi, Balaustium leanderi;*
x) *Stigmaeidae* such as from the genus *Agistemus* e.g. *Agistemus exsertus* Gonzalez, or from the genus *Zetzellia* e.g. *Zetzellia mali* (Ewing)
xi) *Anystidae,* such as from the genus *Anystis,* e.g. *Anystis baccarum.*

3. System according to claim 1, wherein the beneficial mite species is selected from a mite species from the suborder Astigmata such as a mite species selected from:
i) *Carpoglyphidae* such as from the genus *Carpoglyphus* e.g. *Carpoglyphus lactis;*
ii) *Pyroglyphidae* such as from the genus *Dermatophagoides* e.g. *Dermatophagoides pteronysinus, Dermatophagoides farinae;* from the genus *Euroglyphus* e.g. *Euroglyphus longior, Euroglyphus maynei;* from the genus *Pyroglyphus* e.g. *Pyroglyphus africanus;*
iii) *Glycyphagidae* such as from the subfamily *Ctenoglyphinae,* such as from the genus *Diamesoglyphus* e.g. *Diamesoglyphus intermediusor* from the genus *Ctenoglyphus,* e.g. *Ctenoglyphus plumiger, Ctenoglyphus canestrinii, Ctenoglyphus palmifer;* the subfamily *Glycyphaginae,* such as from the genus *Blomia,* e.g. *Blomia freemani* or from the genus *Glycyphagus,* e.g. *Glycyphagus ornatus, Glycyphagus bicaudatus, Glycyphagus privatus, Glycyphagus domesticus,* or from the genus *Lepidoglyphus* e.g. *Lepidoglyphus michaeli, Lepidoglyphus fustifer, Lepidoglyphus destructor,* or from the genus *Austroglycyphagus,* e.g. *Austroglycyphagus geniculatus;* from the subfamily *Aeroglyphinae,* such as from the genus *Aeroglyphus,* e.g. *Aeroglyphus robustus;* from the subfamily *Labidophorinae,* such as from the genus *Gohieria,* e.g. *Gohieria. fusca;* or from the subfamily *Nycteriglyphinae* such as from the genus *Coproglyphus,* e.g. *Coproglyphus stammeri* or from the subfamily *Chortoglyphidae,* such as the genus *Chortoglyphus* e.g. *Chortoglyphus arcuatus* and more preferably is selected from the subfamily *Glycyphaginae,* more preferably is selected from the genus *Glycyphagus* or the genus *Lepidoglyphus* most preferably selected from *Glycyphagus domesticus or Lepidoglyphus destructor;*
iv) *Acaridae* such as from the genus *Tyrophagus* e.g. *Tyrophagus putrescentiae, Tyrophagus tropicus,* from the genus *Acarus* e.g. *Acarus siro, Acarus farris, Acarus gracilis*; from the genus *Lardoglyphus* e.g. *Lardoglyphus konoi,* from the genus *Thyreophagus,* such as *Thyreophagus entomophagus;* from the genus *Aleuroglyphus,* e.g. *Aleuroglyphus ovatus;*
v) *Suidasiidae* such as from the genus *Suidasia,* such as *Suidasia nesbiti, Suidasia pontifica or Suidasia medanensis.*

4. System according to any of the claims 1-2, wherein the beneficial mite species is a predatory mite species and the food source for the predatory mite species comprises a prey mite species selected from the suborder Astigmata.

5. Use of the system according to any of the claims 1-4 for introducing a beneficial mite species in a target area.

6. Method for controlling a pest susceptible of being preyed by a predatory arthropod species comprising providing a number of systems according to claim 1 to a target area where the pest is to be controlled, wherein the predatory arthropod species is a predatory mite species and said system according to claim 1 comprises a population of said predatory mite species, or wherein the predatory arthropod species is present in the target area and said system according to claim 1 comprises a population of an Astigmatid mite species suitable as prey for the predatory arthropod species.

7. Method for producing an agricultural product from non-human organisms prone to infestation by a pest susceptible of being preyed by a predatory arthropod species, said method comprising:
- providing the number of non-human organisms in an area, the target area;
- providing in or in the proximity of the target area a number of systems according to claim 1;
- providing to the number of non-human organisms suitable nutrients and environmental conditions to produce the agricultural product;
wherein the predatory arthropod species is a predatory mite species and said system according to claim 1 comprises a population of said predatory mite species, or wherein the predatory arthropod species is present in the target area and said system according to claim 1 comprises a population of an Astigmatid mite species suitable as prey for the predatory arthropod species.

8. Method according to claim 7, wherein the number of non-human organisms are selected from a crop species, an avian species, preferably a poultry species, mammalian livestock.

## Patentansprüche

1. System zum Freisetzen nützlicher Milben, umfassend ein Abteil, das Milbenabteil, welches eine Population einer nützlichen Milbenspezies, bevorzugterweise in Assoziation mit einem Träger, und eine Nahrungsquelle für die nützlichen Milben beinhaltet, wobei das Milbenabteil von Material, Gassperrenmaterial mit einer Wasserdampfdurchlässigkeitsrate von ≤ 5 g/m²*24 Stunden, umschlossen ist, wobei das Milbenabteil ein Volumen von x mm³ aufweist, wobei x zwischen 3*10³ bis 600*10³ mm³ beträgt, und wobei das System ferner mehrere Verbindungseinrichtungen umfasst, welche das Milbenabteil mit dem Raum außerhalb des Milbenabteils verbinden, wobei die mehreren Verbindungseinrichtungen jeweils eine Fläche y aufweisen, wobei y zwischen 0,1 und 4,0 mm² beträgt, wobei die Summe der Flächen der mehreren Verbindungseinrichtungen Σy ist und wobei 5*10³ mm ≤ x/Σy ≤ 70*10³ mm, vorzugsweise 6*10³ mm ≤ x/Σy ≤ 60*10³ mm, weiter bevorzugt 7*10³ mm ≤ x/Σy ≤ 50*10³ mm.

2. System gemäß Anspruch 1, wobei die nützliche Milbenspezies aus einer Raubmilbenspezies gewählt wird, wie einer Raubmilbenspezies, ausgewählt aus:
- Mesostigmatid-Milbenspezies, wie ausgewählt aus:
*i) Phytoseiidae,* wie aus:
- der Subfamilie der *Amblyseiinae,* wie aus der Gattung *Amblyseius,* z. B. *Amblyseius andersoni, Amblyseius aerialis, Amblyseius swirskii, Amblyseius herbicolus* oder *Amblyseius largoensis,* aus der Gattung *Euseius,* z. B. *Euseius finlandicus, Euseius hibisci, Euseius ovalis, Euseius victoriensis, Euseius stipulatus, Euseius scutalis, Euseius tularensis, Euseius addoensis, Euseius concordis, Euseius ho, Euseius gallicus, Euseius citrifolius* oder *Euseius citri,* aus der Gattung *Iphiseiodes,* z. B. *Iphiseiodes zuluagi,* aus der Gattung *Iphiseius,* z. B. *Iphiseius degenerans,* aus der Gattung *Neoseiulus,* z. B. *Neoseiulus barkeri, Neoseiulus californicus, Neoseiulus cucumeris, Neoseiulus longispinosus, Neoseiulus womersleyi, Neoseiulus idaeus, Neoseiulus anonymus, Neoseiulus paspalivorus, Neoseiulus reductus* oder *Neoseiulus fallacis, Neoseiulus baraki,* aus der Gattung *Amblydromalus,* z. B. *Amblydromalus limonicus,* aus der Gattung *Typhlodromalus,* z. B. *Typhlodromalus aripo, Typhlodromalus lailae* oder *Typhlodromalus peregrinus,* aus der Gattung *Transeius,* z. B. *Transeius montdorensis,* aus der Gattung *Phytoseiulus,* z. B. *Phytoseiulus persimilis, Phytoseiulus macropilis, Phytoseiulus longipes, Phytoseiulus fragariae;*
- der Subfamilie der *Typhlodrominae,* wie aus der Gattung *Galendromus,* z. B. *Galendromus occidentalis,* aus der Gattung *Metaseiulus,* z. B. *Metaseiulus flumenis,* aus der Gattung *Gynaeseiu,* z. B. *Gynaeseius liturivorus,* aus der Gattung *Typhlodromus,* z. B. *Typhlodromus exhilarates, Typhlodromus phialatus, Typhlodromus recki, Typhlodromus transvaalensis, Typhlodromus pyri, Typhlodromus doreenae* oder *Typhlodromus athiasae;*
ii) *Ascidae,* wie aus der Gattung *Proctolaelaps,* wie *Proctolaelaps pygmaeus* (Muller), aus der Gattung *Blattisocius,* z. B. *Blattisocius tarsalis* (Berlese), *Blattisocius keegani* (Fox), aus der Gattung *Lasioseius,* z. B. *Lasioseius fimetorum* Karg, *Lasioseius floridensis* Berlese, *Lasioseius bispinosus* Evans, *Lasioseius dentatus* Fox, *Lasioseius scapulatus* (Kenett), *Lasioseius athiasae* Nawar & Nasr, aus der Gattung *Arctoseius,* z. B. *Arctoseius semiscissus* (Berlese), aus der Gattung *Protogamasellus,* z. B. *Protogamasellus dioscorus* Manson;
iii) *Laelapidae,* wie aus der Gattung *Stratiolaelaps,* z. B. *Stratiolaelaps scimitus* (Womersley), aus der Gattung *Gaeolaelaps,* z. B. *Gaeolaelaps aculeifer* (Canestrini), aus der Gattung *Androlaelaps,* z. B. *Androlaelaps casalis* (Berlese), aus der Gattung *Cosmolaelaps,* z. B. *Cosmolaelaps claviger, Cosmolaelaps jaboticabalensis;*
iv) *Macrochelidae,* wie aus der Gattung *Macrocheles,* z. B. *Macrocheles robustulus* (Berlese), *Macrocheles muscaedomesticae* (Scopoli), *Macrocheles matrius* (Hull);
v) *Parasitidae,* wie aus der Gattung *Pergamasus,* z. B. *Pergamasus quisquiliarum* Canestrini, aus der Gattung *Parasitus,* z. B. *Parasitus fimetorum* (Berlese), *Parasitus bituberosus, Parasitus mycophilus, Parasitus mammilatus;*
- *Prostigmatid-Milbenspezies,* wie aus:
vi) *Tydeidae,* wie aus der Gattung *Homeopronematus,* z. B. *Homeopronematus anconai* (Baker), aus der Gattung *Tydeus,* z. B. *Tydeus lambi* (Baker), *Tydeus caudatus* (Duges), aus der Gattung *Pronematus,* z. B. *Pronematus ubiquitous* (McGregor);
vii) *Cheyletidae,* wie aus der Gattung *Cheyletus,* z. B. *Cheyletus eruditus* (Schrank), *Cheyletus malaccensis* Oudemans;
viii) *Cunaxidae,* wie aus der Gattung *Coleoscirus,* z. B. *Coleoscirus simplex* (Ewing), aus der Gattung *Cunaxa,* z. B. *Cunaxa setirostris* (Hermann);
ix) *Erythraeidae,* wie aus der Gattung *Balaustium,* z. B. *Balaustium putmani* Smiley, *Balaustium medicagoense* Meyer & Ryke, *Balaustium murorum* (Hermann), *Balaustium hernandezi, Balaustium leanderi;*
x) *Stigmaeidae,* wie aus der Gattung *Agistemus,* z. B. *Agistemus exsertus* Gonzalez, oder aus der Gattung *Zetzellia,* z. B. *Zetzellia mali* (Ewing);
xi) *Anystidae,* wie aus der Gattung *Anystis,* z. B. *Anystis baccarum.*

3. System gemäß Anspruch 1, wobei die nützliche Milbenspezies ausgewählt wird aus einer Milbenspezies aus der Unterordnung Astigmata, wie eine Milbenspezies, ausgewählt aus:
i) *Carpoglyphidae,* wie aus der Gattung *Carpoglyphus,* z. B. *Carpoglyphus lactis;*
ii) *Pyroglyphidae,* wie aus der Gattung *Dermatophagoides,* z. B. *Dermatophagoides pteronysinus, Dermatophagoides farinae;* aus der Gattung *Euroglyphus,* z. B. *Euroglyphus longior, Euroglyphus maynei;* aus der Gattung *Pyroglyphus,* z. B. *Pyroglyphus africanus;*
iii) *Glycyphagidae,* wie aus der Subfamilie *Ctenoglyphinae,* wie aus der Gattung *Diamesoglyphus,* z. B. *Diamesoglyphus intermedius,* oder aus der Gattung *Ctenoglyphus,* z. B. *Ctenoglyphus plumiger, Ctenoglyphus canestrinii, Ctenoglyphus palmifer;* der Subfamilie *Glycyphaginae,* wie aus der Gattung *Blomia,* z. B. *Blomia freemani,* oder aus der Gattung *Glycyphagus,* z. B. *Glycyphagus ornatus, Glycyphagus bicaudatus, Glycyphagus privatus, Glycyphagus domesticus,* oder aus der Gattung *Lepidoglyphus,* z. B. *Lepidoglyphus michaeli, Lepidoglyphus fustifer, Lepidoglyphus destructor,* oder aus der Gattung *Austroglycyphagus,* z. B. *Austroglycyphagus geniculatus;* aus der Subfamilie *Aeroglyphinae,* wie aus der Gattung *Aeroglyphus,* z. B. *Aeroglyphus robustus;* aus der Subfamilie *Labidophorinae,* wie aus der Gattung *Gohieria,* z. B. *Gohieria. fusca;* oder aus der Subfamilie *Nycteriglyphinae,* wie aus der Gattung *Coproglyphus,* z. B. *Coproglyphus stammeri,* oder aus der Subfamilie *Chortoglyphidae,* wie der Gattung *Chortoglyphus,* z. B. *Chortoglyphus arcuatus,* und weiter bevorzugt aus der Subfamilie *Glycyphaginae* ausgewählt wird, weiter bevorzugt aus der Gattung *Glycyphagus* oder der Gattung *Lepidoglyphus* ausgewählt wird, am meisten bevorzugt aus *Glycyphagus domesticus* oder *Lepidoglyphus destructor* ausgewählt wird;
iv) *Acaridae,* wie aus der Gattung *Tyrophagus,* z. B. *Tyrophagus putrescentiae, Tyrophagus tropicus,* aus der Gattung *Acarus,* z. B. *Acarus siro, Acarus farris, Acarus gracilis;* aus der Gattung *Lardoglyphus,* z. B. *Lardoglyphus konoi,* aus der Gattung *Thyreophagus,* wie *Thyreophagus entomophagus;* aus der Gattung *Aleuroglyphus,* z. B. *Aleuroglyphus ovatus;*
v) *Suidasiidae,* wie aus der Gattung *Suidasia,* wie *Suidasia nesbiti, Suidasia pontifica* oder *Suidasia medanensis.*

4. System gemäß beliebigen der Ansprüche 1-2, wobei die nützliche Milbenspezies eine Raubmilbenspezies ist, und die Nahrungsquelle für die Raubmilbenspezies eine Beutemilbenspezies umfasst, die aus der Unterordnung Astigmata ausgewählt ist.

5. Verwendung des Systems gemäß beliebigen der Ansprüche 1-4 zum Einbringen einer nützlichen Milbenspezies in ein Zielgebiet.

6. Verfahren zum Bekämpfen eines Schädlings, der dafür anfällig ist, von einer Raubarthropodenspezies als Beute verzehrt zu werden, umfassend das Bereitstellen einer Anzahl von Systemen gemäß Anspruch 1 in einem Zielgebiet, wo der Schädling bekämpft werden soll, wobei die Raubarthropodenspezies eine Raubmilbenspezies ist und das System gemäß Anspruch 1 eine Population der Raubmilbenspezies umfasst, oder wobei die Raubarthropodenspezies im Zielgebiet vorhanden ist und das System gemäß Anspruch 1 eine Population einer als Beute für die Raubarthropodenspezies geeigneten Astigmatid-Milbenspezies umfasst.

7. Verfahren zum Herstellen eines landwirtschaftlichen Produkts aus nichtmenschlichen Organismen, die zu einem Befall durch einen Schädling neigen, der dafür anfällig ist, von einer Raubarthropodenspezies als Beute verzehrt zu werden, wobei das Verfahren umfasst:
- Bereitstellen der Anzahl von nichtmenschlichen Organismen in einem Gebiet, dem Zielgebiet;
- Bereitstellen einer Anzahl von Systemen gemäß Anspruch 1 in dem Zielgebiet oder in der Nähe davon;
- Bereitstellen geeigneter Nährstoffe und Umweltbedingungen an die Anzahl von nichtmenschlichen Organismen, um das landwirtschaftliche Produkt herzustellen;
wobei die Raubarthropodenspezies eine Raubmilbenspezies ist und das System gemäß Anspruch 1 eine Population der Raubmilbenspezies umfasst, oder wobei die Raubarthropodenspezies im Zielgebiet vorhanden ist und das System gemäß Anspruch 1 eine Population einer als Beute für die Raubarthropodenspezies geeigneten Astigmatid-Milbenspezies umfasst.

8. Verfahren gemäß Anspruch 7, wobei die Anzahl von nichtmenschlichen Organismen aus einer Nutzpflanzenspezies, einer Vogelspezies, vorzugsweise einer Geflügelspezies, bzw. Säugetier-Nutzvieh ausgewählt wird.

## Revendications

1. Système destiné à la libération d'acariens bénéfiques comprenant un compartiment, le compartiment d'acariens, contenant une population d'une espèce d'acarien bénéfique, préférablement en association avec un véhicule, et une source alimentaire pour les acariens bénéfiques, où ledit compartiment d'acariens est renfermé par un matériau, un matériau de barrière aux gaz, ayant un taux de transmission de vapeur d'eau ≤ 5 g/m²×24 heures, ledit compartiment d'acariens ayant un volume de x mm³, où x va de 3×10³ à 600×10³ mm³ et où le système comprend en outre un certain nombre de connexions qui relient le compartiment d'acariens à l'espace hors du compartiment d'acariens, ledit nombre de connexions ayant chacune une surface y, où y est compris entre 0,1 et 4,0 mm², où la somme des surfaces du nombre de connexions est Σy et où 5×10³ mm ≤ x/Σy ≤ 70×10³ mm, préférablement 6×10³ mm ≤ x/Σy ≤ 60×10³ mm, plus préférablement 7×10³ mm ≤ x/Σy ≤ 50×10³ mm.

2. Système selon la revendication 1, dans lequel l'espèce d'acarien bénéfique est choisie parmi une espèce d'acarien prédateur, telle qu'une espèce d'acarien prédateur choisie parmi :
- une espèce d'acarien *Mesostigmata* choisie parmi :
i) *Phytoseiidae* tel que parmi :
- la sous-famille des *Amblyseiinae,* tel que du genre *Amblyseius,* par exemple *Amblyseius andersoni, Amblyseius aerialis, Amblyseius swirskii, Amblyseius herbicolus* ou *Amblyseius largoensis,* du genre *Euseius* par exemple *Euseius finlandicus, Euseius hibisci, Euseius ovalis, Euseius victorien sis, Euseius stipulatus, Euseius scutalis, Euseius tularensis, Euseius addoensis, Euseius concordis, Euseius ho, Euseius gallicus, Euseius citrifolius* ou *Euseius citri,* du genre *Iphiseiodes* par exemple *Iphiseiodes zuluagi,* du genre *Iphiseius* par exemple *Iphiseius degenerans,* du genre *Neoseiulus* par exemple *Neoseiulus barkeri, Neoseiulus californicus, Neoseiulus cucumeris, Neoseiulus longispinosus, Neoseiulus womersleyi, Neoseiulus idaeus, Neoseiulus anonymus, Neoseiulus paspalivorus, Neoseiulus reductus* ou *Neoseiulus fallacis, Neoseiulus baraki,* du genre *Amblydromalus* par exemple *Amblydromalus limonicus,* du genre *Typhlodromalus* par exemple *Typhlodromalus aripo, Typhlodromalus laila* ou *Typhlodromalus peregrinus,* du genre *Transeius* par exemple *Transeius montdorensis,* du genre *Phytoseiulus,* par exemple *Phytoseiulus persimilis, Phytoseiulus macropilis, Phytoseiulus longipes, Phytoseiulus fragariae ;*
- la sous-famille des *Typhlodrominae,* tel que du genre *Galendromus* par exemple *Galendromus occidentalis,* du genre *Metaseiulus* par exemple *Metaseiulus flumenis,* du genre *Gynaeseius* par exemple *Gynaeseius liturivorus,* du genre *Typhlodromus* par exemple *Typhlodromus exhilarates, Typhlodromus phialatus, Typhlodromus recki, Typhlodromus transvaalensis, Typhlodromus pyri, Typhlodromus doreenae* ou *Typhlodromus athiasae ;*
ii) *Ascidae* tel que du genre *Proctolaelaps,* tel que *Proctolaelaps pygmaeus* (Muller) ; du genre *Blattisocius* par exemple *Blattisocius tarsalis* (Berlese), *Blattisocius keegani* (Fox) ; du genre *Lasioseius* par exemple *Lasioseius fimetorum* Karg, *Lasioseius floridensis* Berlese, *Lasioseius bispinosus* Evans, *Lasioseius dentatus* Fox, *Lasioseius scapulatus* (Kenett), *Lasioseius athiasae* Nawar & Nasr ; du genre *Arctoseius* par exemple *Arctoseius semiscissus* (Berlese) ; du genre *Protogamasellus* par exemple *Protogamasellus dioscorus* Manson ;
iii) *Laelapidae* tel que du genre *Stratiolaelaps* par exemple *Stratiolaelaps scimitus* (Womersley) ; du genre *Geolaelaps* par exemple *Geolaelaps aculeifer* (Canestrini) ; du genre *Androlaelaps* par exemple *Androlaelaps casalis* (Berlese) ; du genre *Cosmolaelaps* par exemple *Cosmolaelaps claviger, Cosmolaelaps jaboticabalensis ;*
iv) *Macrochelidae* tel que du genre *Macrocheles* par exemple *Macrocheles robustulus* (Berlese), *Macrocheles muscaedomesticae* (Scopoli), *Macrocheles matrius* (Hull) ;
v) *Parasitidae* tel que du genre *Pergamasus* par exemple *Pergamasus quisquiliarum* Canestrini ; du genre *Parasitus* par exemple *Parasitus fimetorum* (Berlese), *Parasitus bituberosus ; Parasitus mycophilus, Parasitus mammillatus ;*
- une espèce d'acarien *Prostigmata,* tel que parmi:
vi) *Tydeidae* tel que du genre *Homeopronematus* par exemple *Homeopronematus anconai* (Baker) ; du genre *Tydeus* par exemple *Tydeus lambi* (Baker), *Tydeus caudatus* (Dugés), du genre *Pronematus* par exemple *Pronematus ubiquitous* (McGregor) ;
vii) *Cheyletidae* tel que du genre *Cheyletus* par exemple *Cheyletus eruditus* (Schrank), *Cheyletus malaccensis* Oudemans ;
viii) *Cunaxidae* tel que du genre *Coleoscirus* par exemple *Coleoscirus simplex* (Ewing), du genre *Cunaxa* par exemple *Cunaxa setirostris* (Hermann) ;
ix) *Erythraeidae* tel que du genre *Balaustium* par exemple *Balaustium putmani* Smiley, *Balaustium medicagoense* Meyer & Ryke, *Balaustium murorum* (Hermann) ; *Balaustium hernandezi, Balaustium leanderi ;*
x) *Stigmaeidae* tel que du genre *Agistemus* par exemple *Agistemus exsertus* Gonzalez ; ou du genre *Zetzellia* par exemple *Zetzellia mali* (Ewing) ;
xi) *Anystidae,* tel que du genre *Anystis,* par exemple *Anystis baccarum.*

3. Système selon l'une quelconque des revendications 1, dans lequel l'espèce d'acarien bénéfique est choisie parmi une espèce d'acarien issue du sous-ordre *Astigmata,* telle qu'une espèce d'acarien choisie parmi :
i) *Carpoglyphidae* tel que du genre *Carpoglyphus* par exemple *Carpoglyphus lactis ;*
ii) *Pyroglyphidae* tel que du genre *Dermatophagoides* par exemple *Dermatophagoides pteronyssinus, Dermatophagoides farinae* ; du genre *Euroglyphus* par exemple *Euroglyphus longior, Euroglyphus maynei* ; du genre *Pyroglyphus* par exemple *Pyroglyphus africanus ;*
iii) *Glycyphagidae* tel que de la sous-famille *Ctenoglyphinae,* tel que du genre *Diamesoglyphus* par exemple *Diamesoglyphus intermedius* ou du genre *Ctenoglyphus,* par exemple *Ctenoglyphus plumiger, Ctenoglyphus canestrinii, Ctenoglyphus palmifer* ; la sous-famille *Glycyphaginae,* tel que du genre *Blomia,* par exemple *Blomia freemani* ou du genre *Glycyphagus,* par exemple *Glycyphagus ornatus, Glycyphagus bicaudatus, Glycyphagus privatus, Glycyphagus domesticus,* ou du genre *Lepidoglyphus* par exemple *Lepidoglyphus michaeli, Lepidoglyphus fustifer, Lepidoglyphus destructor,* ou du genre *Austroglycyphagus,* par exemple *Austroglycyphagus geniculatus* ; de la sous-famille *Aeroglyphinae,* tel que du genre *Aeroglyphus,* par exemple *Aeroglyphus robustus* ; de la sous-famille *Labidophorinae,* tel que du genre *Gohieria,* par exemple *Gohieria fusca* ; ou de la sous-famille *Nycteriglyphinae* tel que du genre *Coproglyphus,* par exemple *Coproglyphus stammeri* ou de la sous-famille *Chortoglyphidae,* tel que du genre *Chortoglyphus* par exemple *Chortoglyphus arcuatus* et plus préférablement est choisi dans la sous-famille *Glycyphaginae,* plus préférablement est choisi dans le genre *Glycyphagus* ou le genre *Lepidoglyphus,* tout préférablement choisi parmi *Glycyphagus domesticus* ou *Lepidoglyphus destructor ;*
iv) *Acaridae* tel que du genre *Tyrophagus* par exemple *Tyrophagus putrescentiae, Tyrophagus tropicus,* du genre *Acarus* par exemple *Acarus siro, Acarus farris, Acarus gracilis* ; du genre *Lardoglyphus* par exemple *Lardoglyphus konoi,* du genre *Thyreophagus,* tel que *Thyreophagus entomophagus* ; du genre *Aleuroglyphus,* par exemple *Aleuroglyphus ovatus ;*
v) *Suidasiidae* tel que du genre *Suidasia,* tel que *Suidasia nesbiti, Suidasia pontifica* ou *Suidasia medanensis.*

4. Système selon l'une quelconque des revendications 1-2, dans lequel l'espèce d'acarien bénéfique est une espèce d'acarien prédateur et la source alimentaire pour l'espèce d'acarien prédateur comprend une espèce d'acarien proie choisie parmi le sous-ordre *Astigmata.*

5. Utilisation du système selon l'une quelconque des revendications 1-4, pour l'introduction d'une espèce d'acarien bénéfique dans une zone cible.

6. Méthode de contrôle d'un nuisible susceptible d'être la proie d'une espèce d'arthropode prédateur, comprenant la fourniture d'un certain nombre de systèmes selon la revendication 1 à une zone cible où le nuisible doit être contrôlé, où l'espèce d'arthropode prédateur est une espèce d'acarien prédateur et ledit système selon la revendication 1 comprend une population de ladite espèce d'acarien prédateur, ou où l'espèce d'arthropode prédateur est présente dans la zone cible et ledit système selon la revendication 1 comprend une population d'une espèce d'acarien *Astigmata* convenable comme proie pour l'espèce d'arthropode prédateur.

7. Méthode de production d'un produit agricole à partir d'organismes non humains prédisposés à une infestation par un nuisible susceptible d'être une proie pour une espèce d'arthropode prédateur, ladite méthode comprenant :
- la fourniture du nombre d'organismes non humains dans une zone, la zone cible ;
- la fourniture dans, ou à proximité de, la zone cible, d'un certain nombre de systèmes selon la revendication 1 ;
- la fourniture au nombre d'organismes non humains de nutriments ainsi que de conditions environnementales convenables afin de produire le produit agricole ;
où l'espèce d'arthropode prédateur est une espèce d'acarien prédateur et ledit système selon la revendication 1 comprend une population de ladite espèce d'acarien prédateur, ou où l'espèce d'arthropode prédateur est présente dans la zone cible et ledit système selon la revendication 1 comprend une population d'une espèce d'acarien *Astigmata* convenable comme proie pour l'espèce d'arthropode prédateur.

8. Méthode selon la revendication 7, dans laquelle le nombre d'organismes non humains est choisi parmi une espèce de culture, une espèce aviaire, préférablement une espèce de volaille, un élevage mammalien.
